(19) Europäisches Patentamt
European Patent Office
Office européen des brevets

(11) EP 3 917 589 B1

(12) **EUROPEAN PATENT SPECIFICATION**

(45) Date of publication and mention of the grant of the patent:
**11.09.2024 Bulletin 2024/37**

(21) Application number: **20704750.7**

(22) Date of filing: **16.01.2020**

(51) International Patent Classification (IPC):
***A61M 1/00*** *(2006.01)*

(52) Cooperative Patent Classification (CPC):
**A61M 1/74; A61M 1/96; A61M 1/966;**
A61M 2205/15; A61M 2205/18; A61M 2205/3344;
A61M 2205/52; A61M 2205/8212

(86) International application number:
**PCT/US2020/013807**

(87) International publication number:
**WO 2020/159713 (06.08.2020 Gazette 2020/32)**

(54) **CONTROL ALGORITHM FOR NEGATIVE PRESSURE WOUND THERAPY DEVICES**

REGELUNGSALGORITHMUS FÜR UNTERDRUCK-THERAPIEGERÄTE

ALGORITHME DE COMMANDE POUR DISPOSITIFS DE THÉRAPIE DE PLAIE À PRESSION NÉGATIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2019 US 201962797480 P**

(43) Date of publication of application:
**08.12.2021 Bulletin 2021/49**

(73) Proprietor: **Solventum Intellectual Properties Company**
**Maplewood, MN 55144 (US)**

(72) Inventors:
- **SEDDON, James**
  **San Antonio, Texas 78265 (US)**
- **PRATT, Benjamin A.**
  **San Antonio, Texas 78265 (US)**

(74) Representative: **Simmons & Simmons**
**City Point**
**One Ropemaker Street**
**London EC2Y 9SS (GB)**

(56) References cited:
**WO-A1-2013/140255 WO-A1-2018/041854**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 917 589 B1

## Description

BACKGROUND

The present disclosure relates generally to negative pressure wound therapy (NPWT) devices and more particularly control algorithms for NPWT devices. It would be desirable to provide a NPWT device which can identify leaks, adaptively operate despite the leaks, and can optimize negative pressure setpoints to optimize battery usage.

WO2018/041854A1 discloses a negative pressure apparatus which includes a negative pressure source configured to provide negative pressure via a fluid flow path to a wound dressing placed to create a seal over a wound, a pressure sensor, and a controller. The controller can be configured to operate the negative pressure source in a first mode and determine a change in pressure in the fluid flow path over a period of time based on a plurality of measurements by the pressure sensor. In response to a determination that pressure in the fluid flow path is decreasing, the controller can operate the negative pressure source in a second mode in which greater amount of negative pressure is provided than in the first mode. In response to a determination that pressure in the fluid flow path is not decreasing, the controller can provide an indication of a first leak in the seal.

WO2013/140255A1 discloses a controller configured to monitor a duty cycle of a source of negative pressure in a negative pressure wound therapy apparatus. Based on the monitored duty cycle, the controller can determine whether a leak is present and provide an indication to a user. The controller can determine a duty cycle threshold in order to achieve an optimal or near optimal balance between an uninterrupted delivery of therapy, avoidance inconveniencing a user, conserving power, achieving optimal or near optimal efficiency, and/or limiting vibrational noise. In some embodiments, the duty cycle threshold is determined based at least in part on a capacity of a power source and an operational time of the apparatus.

SUMMARY

One implementation of the present disclosure is a negative pressure wound therapy (NPWT) device. The NPWT device is configured to perform NPWT and includes a battery configured to supply the NPWT device with power, a pump configured to receive power from the battery and to produce a vacuum at a setpoint pressure to perform the NPWT, a user interface configured to provide alerts to a user, and a controller configured to receive power from the battery and to adjust the setpoint pressure of the pump, according to some embodiments. The controller is configured to operate the NPWT device in a standard therapy mode, a seal assist therapy mode, a pressure optimization mode, and a preservation mode of operation, according to some embodiments. The standard therapy mode includes operating the pump at a first setpoint vacuum pressure and periodically comparing a determined duty cycle value of the pump to a predetermined duty cycle threshold value, according to some embodiments. The seal assist therapy mode includes operating the pump at a second setpoint vacuum pressure, according to some embodiments. The second setpoint vacuum pressure is greater than the first setpoint vacuum pressure. The pressure optimization mode includes determining an initial pump duty cycle value and an initial battery capacity, reducing the setpoint pressure of the vacuum by a determined amount at an end of a timestep, determining a second pump duty cycle value at the end of the timestep, monitoring an actual vacuum pressure produced by the pump at the end of the timestep, and repeating the steps of reducing the setpoint pressure, calculating the second pump duty cycle value, and monitoring the actual vacuum pressure of the pump until the second pump duty cycle value is less than the predetermined duty cycle threshold value. The determined amount and the timestep are determined based on at least one of the initial pump duty cycle value and the initial battery capacity. The preservation mode of operation includes reducing the setpoint vacuum pressure to an absolute minimum pressure. Optional features are set out in the dependent claims.

BRIEF DESCRIPTION OF THE DRAWINGS

FIG. 1 is a front view of a NPWT device shown to include a user interface and a controller, according to an exemplary embodiment.

FIG. 2 is a block diagram of the controller of FIG. 1, according to an exemplary embodiment.

FIG. 3 is an illustrative graph of a duty cycle of a pump of the NPWT device of FIG. 1, according to an exemplary embodiment.

FIG. 4 is a block diagram of a control algorithm of the NPWT device of FIG. 1, according to an exemplary embodiment.

FIG. 5a is a block diagram flow chart of a control algorithm for the NPWT device of FIG. 1, shown to include a standard therapy mode and a seal assist mode, according to an exemplary embodiment.

FIG. 5b is a block diagram flow chart of the control algorithm of FIG. 5a for the NPWT device of FIG. 1, shown to include a pressure optimization mode, according to an exemplary embodiment.

FIG. 5c is a block diagram flow chart of the control algorithm of FIG. 5a for the NPWT device of FIG. 1, shown to include a preservation mode, according to an exemplary embodiment.

FIG. 6a is a block diagram flow chart of the control algorithm of FIG. 5a for the NPWT device of FIG. 1, shown to include an alternative pressure optimization mode, according to an exemplary embodiment.

FIG. 6b is a block diagram flow chart of the control algorithm of FIG. 5a for the NPWT device of FIG. 1, shown to include an alternative preservation mode, according to an exemplary embodiment.

DETAILED DESCRIPTION

## Overview

Referring generally to the FIGURES, a control algorithm for a NPWT device is shown, according to some embodiments. In some embodiments, the control algorithm includes a standard therapy pressure mode, a seal assist mode, a pressure optimization mode, and a preservation mode. The control algorithm switches the NPWT device between any of these modes based on various conditions, according to some embodiments. In some embodiments, the NPWT device is switched between these modes based at least one of a determination that a leak has occurred, a pump duty cycle value exceeding a threshold, and an energy/charge level of a power source. The control algorithm identifies leak events, and attempts to seal the leak by increasing setpoint pressure (seal assist mode), according to some embodiments. If the leak cannot be sealed with the seal assist mode, the NPWT device transitions into the pressure optimization mode where therapy is continued and the control algorithm attempts to provide as much negative pressure as possible given the constraints of the leakage amount and the energy/charge level of the power source. The control algorithm may adaptively increase or decrease the setpoint pressure to determine the optimal setpoint pressure, according to some embodiments. In some embodiments, the control algorithm increases or decreases the setpoint pressure linearly. In some embodiments, the control algorithm increases or decreases the setpoint pressure non-linearly based on time and/or pressure. Alternatively, the control algorithm may gradually reduce the pressure stepping to maintain higher therapy pressures for as long as possible while using lower target pressure/reduced pump duties to validate a duration of each pressure increment for gradually longer (as power consumption is reduced during each pressure lowering). In some embodiments, if the setpoint pressure cannot be maintained above a threshold value and/or if the energy/charge level of the power source drops below a threshold value, the control algorithm may transition the NPWT device into the preservation mode. The preservation mode provides an alert to a user (e.g., the patient) and attempts to provide a minimal amount of negative pressure, working on the basis that some negative pressure is better than none. Advantageously, the control algorithm allows a continuation of therapy despite a persistent dressing leak, reduces the need for the user to replace the power source (e.g., battery cells) early due to the persistent dressing leak, and provides fewer device alarms (e.g., low battery, leakage event, etc.). Additionally, the control algorithm conserves the energy/charge level of the power source (e.g., battery life) by reducing the setpoint pressure when a leak is detected and preventing the NPWT device from wasting the energy/charge of the power source.

## NPWT Device

Referring now to FIG. 1, a front view of a NPWT device 100 is shown, according to an exemplary embodiment. The NPWT device 100 includes a user interface 106, buttons 104, a housing 102, and a controller 110, according to some embodiments. In some embodiments, controller 110 is configured to control an operation of pump 142 to perform a NPWT. In some embodiments, NPWT device 100 is configured to control an operation of a V.A.C. VERAFLO™ Therapy, a PREVENA™ Therapy, an ABTHERA™ Open Abdomen Negative Pressure Therapy, or any other NPWT (e.g., controller 110 is configured to adjust an operation of pump 142 to perform any of the herein mentioned NPWT). In some embodiments, NPWT device 100 is configured to control an operation of any devices necessary to complete any of the herein mentioned NPWT (e.g., a pump, a vacuum system, an instillation system, etc.). In some embodiments, NPWT device 100 is a disposable NPWT device (dNPWT) and may have reusable/disposable parts. For example, NPWT device 100 may be relatively lightweight (e.g., less than 5 pounds), and may be portable, allowing a patient to transport NPWT device 100 while NPWT device 100 still performs NPWT, according to some embodiments. Since NPWT device 100 may be portable, NPWT device 100 may draw power from a portable power source (e.g., power source 120, a battery, etc.). The portable power source has a limited energy capacity, and therefore optimization of the portable power source is advantageous, since when the portable power source runs out of energy, NPWT can no longer be performed.

User interface 106 is configured to display any of an alarm/alert regarding at least one of a battery capacity of NPWT device 100, a leak, a pump duty cycle, etc., according to some embodiments. In some embodiments, user interface 106 is configured to provide any of a visual and an auditory alert. In some embodiments, user interface 106 allows a user to adjust an operation of the NPWT performed by NPWT device 100. For example, the user may provide a user input to controller 110 through user interface 106 to increase a pressure setpoint of pump 142, adjust a type of NPWT performed, adjust a parameter/operation of the performed NPWT, adjust a duration of the performed NPWT, pause the NPWT, start the NPWT, transition the NPWT device 100 into a "change" mode (e.g., so that wound dressings can be changed), etc. In some embodiments, user interface 106 is any of a resistive touch-screen interface, a surface acoustic wave touch-screen interface, a capacitive touch-screen interface, etc., configured to allow the user to control NPWT device 100. In some embodiments, user interface 106 is controlled by buttons 104. In some embodiments, buttons 104 are configured to control user interface 106 and/or to adjust an operation of the NPWT performed by NPWT device 100.

User interface 106 is also configured to display an operational status of the performed NPWT, according to some embodiments. For example, user interface 106 may display any of a patient name, a responsible caregiver's name, a type of NPWT currently being performed by NPWT device 100, a duration of NPWT, a time remaining in the current NPWT, a vacuum pressure of the NPWT, etc., or any other information relevant to the NPWT and/or operational status of NPWT device 100. For example, user interface 106 is configured to display a remaining battery life of a battery (e.g., power source 120 as shown in FIG. 2), and/or a duty cycle of the system configured to provide vacuum pressure to a wound (e.g., pump 142), according to some embodiments. In some embodiments, the remaining battery life of the battery is a remaining amount of energy in the battery. In some embodiments, the remaining battery life of the battery is a remaining amount of time which NPWT device 100 can sustain NPWT device at a current operational status.

## Controller Configuration

Referring now to FIG. 2, a block diagram of controller 110 used in NPWT device 100 is shown, according to an exemplary embodiment. Controller 110 is configured to control an operation of pump 142 to perform the NWPT, according to some embodiments. In some embodiments, controller 110 is configured to transition NPWT device 100 between various modes of operation (e.g., standard therapy mode, seal assist mode, pressure optimization mode, preservation mode, etc.). Controller 110 is shown to include a processing circuit, shown as processing circuit 112, according to some embodiments. Processing circuit 112 may be configured to perform some or all of the functionality of controller 110. Processing circuit 112 is shown to include a processor, shown as processor 114. Processor 114 may be a general purpose single- or multi-chip processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein. Processor 114 may be a microprocessor, or, any conventional processor, controller, microcontroller, or state machine. Processor 114 also may be implemented as a combination of computing devices, such as a combination of a DSP and a microprocessor, a plurality of microprocessors, one or more microprocessors in conjunction with a DSP core, or any other such configuration. In some embodiments, particular processes and methods may be performed by circuitry that is specific to a given function. Processing circuit 112 also include memory, shown as memory 116. Memory 116 (e.g., memory, memory unit, storage device) may include one or more devices (e.g., RAM, ROM, Flash memory, hard disk storage) for storing data and/or computer code for completing or facilitating the various processes, layers and modules described in the present disclosure. Memory 116 may be or include volatile memory or non-volatile memory, and may include database components, object code components, script components, or any other type of information structure for supporting the various activities and information structures described herein. According to an exemplary embodiment, the memory 116 is communicably connected to the processor 114 via processing circuit 112 and includes computer code for executing (e.g., by the processing circuit or the processor) the one or more processes described herein.

Referring still to FIG. 2, controller 110 is shown to include a power interface, shown as power interface 118, according to an exemplary embodiment. Power interface 118 is configured to draw power supplied by a power source, shown as power source 120, to power controller 110, according to some embodiments. In some embodiments, power source 120 is any kind of permanent and/or temporary power source. In some embodiments, power source 120 is a battery. In some embodiments, power interface 118 is a connection port for a permanent power source (e.g., AC power and/or DC power) such as a wired 24VAC connection. In other embodiments, power interface 118 includes both a port for permanent power and/or a power circuit configured to receive and transform power from power source 120. In some embodiments, power interface 118 is configured to receive power from both a permanent power source (e.g., an outlet) and a temporary power source (e.g., a battery). Power interface 118 may include any number of electrical components such as resistors, transistors, capacitors, inductors, diodes, transformers, transistors, switches, etc., necessary to receive, transform, and supply power to controller 110, according to some embodiments. In some embodiments, if power interface 118 is configured to receive power from a temporary power source (e.g., if power source 120 is a battery), power interface 118 may output power level data of power source 120 to processing circuit 112. The power level data may indicate an amount of energy remaining in power source 120 (e.g., a number of kW-hrs remaining in power source 120). In some embodiments, power source 120 is a replaceable power source (e.g., a battery). In some embodiments, power source 120 is one or more disposable batteries. For example, power source 120 is one or more disposable 12-volt batteries, according to some embodiments. In some embodiments, power source 120 is one or more rechargeable batteries. In some embodiments, power source 120 is configured to be temporarily disconnected from power interface 118 when the replaceable power source must be replaced (e.g., if power source 120 is one or more replaceable batteries, power source 120 may be disconnected when the battery level is low and the batteries must be replaced).

Referring still to FIG. 2, memory 116 is shown to include power source capacity module 130, according to some embodiments. In some embodiments, power source capacity module 130 is configured to receive information from power interface 118 regarding a remaining energy/charge of power source 120. In some embodiments, power source capacity module 130 measures any of a supplied current from power source 120, a voltage from power source 120, and an amount of time power source 120 has provided power to controller 110. Power source capacity module 130 may determine an amount of charge used over the amount of time power source 120 has provided power to controller 110, according to some embodiments. In some embodiments, power capacity module 130 determines an amount of energy used over the amount of time. For example, power capacity module 130 may determine an amount of charge used over a time period (e.g., using $Q = I * t$), and determine a remaining amount of charge of power source 120 based on a difference between a total charge capacity of power source 120 and the amount of charge used over the time period. In response to determining the amount of charge remaining in power source 120, power source capacity module 130 may determine a remaining amount of energy in power source 120 (e.g., by $E = V * Q$). In some embodiments, power capacity module 130 uses supplied voltage from power source 120 to determine a remaining amount of energy in power source 120. Power capacity module 130 may receive an indication of remaining energy (or charge) in power source 120, or may determine remaining energy (or charge) in power source 120, according to some embodiments.

Referring still to FIG. 2, power source capacity module 130 is shown providing mode transition module 132 with an indication of an amount of energy remaining in power source 120, according to some embodiments. For example, power source capacity module 130 may provide mode transition module 132 with any of a charge remaining in power source 120, an amount of energy (e.g., kW-hrs), and a percent of remaining energy and/or charge in power source 120. Power source capacity module 130 provides mode transition module 132 with remaining energy level of power source 120 in a percentage (e.g., 50% charge remaining, 75% charge remaining, etc.), according to some embodiments. Mode transition module 132 uses the indication of energy/charge remaining in power source 120 to determine operational changes of NPWT device 100 and/or operational changes of pump 142, according to some embodiments. In some embodiments, mode transition module 132 is configured to use the indication of remaining energy/charge in power source 120 to determine whether or not to transition NPWT device 100 and/or pump 142 between various modes of operation, described in greater detail below.

Referring still to FIG. 2, controller 110 is shown to include input interface 140 and output interface 138, according to some embodiments. Input interface 140 is configured to receive inputs from at least one of pump 142 and user interface 106, according to some embodiments. In some embodiments, input interface 140 receives commands and/or requests from user interface 106. For example, user interface 106 may receive a command from user interface 106 to transition NPWT device 100 between various modes of operation, or to adjust an operational characteristic of the NPWT being performed by NPWT device 100 (e.g., increasing a pressure setpoint, increasing an amount of therapy time, etc.). Input interface 140 is also configured to receive information from pump 142 regarding an actual therapy pressure, according to some embodiments.

Referring still to FIG. 2, controller 110 is shown to include a pulse width modulation (PWM) module 136, according to some embodiments. PWM module 136 receives a therapy pressure setpoint and performs PWM to adjust a duty cycle of pump 142 to achieve the therapy pressure setpoint, according to some embodiments. PWM module 136 is also shown receiving a feedback (i.e., actual therapy pressure) from pump 142 through input interface 140, according to some embodiments. Pump 142 is configured to provide the therapy pressure to a wound, with a seal being placed between the wound and vacuum tubes used to apply the therapy pressure (e.g., negative pressure) to the wound. The vacuum tubes, wound, and any other vacuum elements used to provide the therapy pressure to the wound may be referred to as the vacuum system, according to some embodiments. The seal between the wound and the vacuum tubes may sometimes leak, causing PWM module 136 to increase the duty cycle of pump 142 to achieve the therapy pressure setpoint (i.e., actual therapy pressure = setpoint therapy pressure). In order to overcome pressure losses due to the leakage, pump 142 must operate at a higher pump duty cycle. In this way, a leak in the vacuum system is positively correlated to the duty cycle required to achieve the therapy pressure setpoint. Therefore, an unusually high pump duty cycle to achieve the therapy pressure setpoint may indicate a leak in the vacuum system, according to some embodiments. In this way, leaks may be identified and alerts may be provided to the user through user interface 106, according to some embodiments. Additionally, the identification of leaks and the corresponding pump duty cycle may be used by mode transition module 132 to determine when to switch from one mode of operation to another mode of operation, according to some embodiments.

Referring still to FIG. 2, controller 110 is shown to include duty cycle module 134, according to some embodiments. Duty cycle module 134 is configured to communicate with PWM module 136 and monitor a current pump duty cycle of PWM module 136, according to some embodiments. In some embodiments, duty cycle module 134 is configured to supply the monitored current pump duty cycle of PWM module 136 to mode transition module 132. In some embodiments, duty cycle module 134 stores historical information of pump duty cycles used by PWM module 136 over a time period, and supplies this historical information to mode transition module 132. For example, duty cycle module 134 may identify and store a maximum pump duty cycle and provide the maximum pump duty cycle to mode transition module 132, according to some embodiments. In some embodiments, duty cycle module 134 may store a pump duty cycle threshold value and compare the monitored pump duty cycle value from PWM module 136 to the pump duty cycle threshold value. In some embodiments, the pump duty cycle threshold value is a predetermined value. In some embodiments, the pump duty cycle threshold value is determined based on at least one of a type of NPWT being performed (e.g., V.A.C. VERAFLO™ Therapy, PREVENA™ Therapy, ABTHERA™ Therapy, etc.) a type of NPWT device (e.g., various models of NPWT device 100), a type of pump 142, a rating of pump 142 (e.g., a particular pump may be rated for a maximum pump duty cycle), a duration of therapy time, an energy capacity of power source 120 (e.g., 100% charge remaining, 50% charge remaining, 50 kW-hrs remaining, etc.), a mode of operation of NPWT device 100 (e.g., standard therapy mode, seal assist mode, etc.), etc. If the pump duty cycle threshold value is determined rather than being a predetermined value, duty cycle module 134 may be configured to determine the pump duty cycle threshold value using any of an equation, a set of equations, a lookup table, a graph, a database, a script object, a function, etc. In some embodiments, duty cycle module 134 periodically receives/monitors pump duty cycle values from PWM module 136 at an end of a time step and periodically provides mode transition module 132 with the periodic pump duty cycle values. In some embodiments, duty cycle module 134 receives pump duty cycle values from PWM module 136 at an end of a time step having a predetermined duration (e.g., 1 second, 5 seconds, 1 minute, etc.).

In some embodiments, duty cycle module 134 is configured to calculate a continuous pump duty value. The continuous pump duty value ensures that the NPWT can be maintained for the prescribed therapy duration, according to some embodiments. Duty cycle module 134 is configured to calculate the continuous pump duty value based on any of a type of NPWT being performed (e.g., V.A.C. VERAFLO™ Therapy, PREVENA™ Therapy, ABTHERA™ Therapy, etc.) a type of NPWT device (e.g., various models of NPWT device 100), a type of pump 142, a rating of pump 142 (e.g., a particular pump may be rated for a maximum pump duty cycle), a duration of therapy time, an energy capacity of power source 120 (e.g., 100% charge remaining, 50% charge remaining, 50 kW-hrs remaining, etc.), a mode of operation of NPWT device 100 (e.g., standard therapy mode, seal assist mode, etc.), etc. Duty cycle module 134 may calculate the continuous pump duty value using any of an equation, a set of equations, a lookup table, a graph, a database, a script object, a function, etc. Duty cycle module 134 is configured to provide mode transition module 132 with the continuous pump duty value, according to some embodiments. In some embodiments, duty cycle module 134 calculates the continuous pump duty cycle value at a beginning of NPWT, while in some embodiments, duty cycle module 134 calculates the continuous pump duty cycle value periodically. In some embodiments, the periodically calculated continuous pump duty cycle value indicates a maximum pump duty cycle value which can be used to still perform the NPWT for the prescribed therapy duration, given current energy/charge level of power source 120. In this way, duty cycle module 134 may provide information to mode transition module whether or not a current pump duty cycle value is tenable to complete the NPWT for the prescribed therapy duration given the current energy/charge capacity of power source 120, according to some embodiments.

Referring still to FIG. 2, controller 110 includes mode transition module 132, according to some embodiments. Mode transition module 132 is configured to adjust a mode of operation of NPWT device 100, according to some embodiments. In some embodiments, mode transition module 132 is configured to transition NPWT device 100 between various predetermined modes of operation. The various predetermined modes of operation are shown a standard therapy mode of operation, a seal assist mode of operation, a pressure optimization mode of operation and a preservation mode of operation. Each of standard therapy module 122, seal assist module 124, pressure optimization module 126, and preservation mode module 128 are shown outputting therapy pressure setpoint values to PWM module 136, according to some embodiments. In some embodiments, mode transition module 132 is configured to determine which of standard therapy module 122, seal assist module 124, pressure optimization module 126, and preservation mode module 128 is allowed to output therapy pressure setpoints to PWM module 136 to adjust operation of NPWT device 100. In some embodiments, standard therapy module 122 is configured to provide PWM module 136 with therapy pressure setpoints to operate according to the standard therapy mode of operation, seal assist module 124 is configured to provide PWM module 136 with therapy pressure setpoints to operate according to the seal assist mode of operation, pressure optimization module 126 is configured to provide PWM module 136 with therapy pressure setpoints to operate according to the pressure optimization mode of operation, and preservation mode module 128 is configured to provide PWM module 136 with therapy pressure setpoints to operate according to the preservation mode of operation. The functionality of each of these modes of operation is described in greater detail below with reference to FIGS. 4-6b, according to some embodiments.

Mode transition module 132 is configured to transition NPWT device 100 between the above mentioned modes of operation, according to some embodiments. Mode transition module 132 is shown receiving input information from any of power source capacity module 130, duty cycle module 134, input interface 140, and any of standard therapy module 122, seal assist module 124, pressure optimization module 126, and preservation mode module 128, according to some embodiments. In some embodiments, mode transition module 132 receives information from any of the above mentioned modules regarding energy/charge remaining in power source 120, therapy pressure setpoint of pump 142, actual therapy pressure of pump 142, user inputs from user interface 106, current pump duty cycle, historical pump duty cycle, continuous pump duty cycle, etc. In some embodiments, mode transition module 132 is configured to determine when to transition NPWT device 100 between any of the predefined modes of operation based on any of the information received as described hereinabove. The methods and functions of how mode transition module 132 determines when to transition NPWT device 100 between the predefined modes of operation is described in greater detail below with reference to FIGS. 4-6b, according to some embodiments.

In some embodiments, mode transition module 132 is also configured to determine when to output an alarm to user interface 106. In this way, mode transition module 132 may act as an alarm/alert module, according to some embodiments. In some embodiments, a separate alarm/alert module is used in conjunction with mode transition module 132 to determine when to output the alarm/alert to user interface 106. Additionally, either of mode transition module 132 or the alarm/alert module may determine a type of alert/alarm to be displayed to the user via user interface 106, according to some embodiments. For example, in some cases, either of mode transition module 132 and the alarm/alert module may determine that a visual alarm/alert should be provided to the user through user interface 106, while in other cases both an auditory and a visual alert should be provided to the user through user interface 106, according to some embodiments. In some embodiments, any of standard therapy module 122, seal assist module 124, pressure optimization module 126, and preservation mode module 128 determine when to provide an alarm/alert to the user through user interface 106, as well as the type of alert to be provided. Any of the modules described hereinabove which may be configured to determine if an alert should be provided, and the type of alert to be provided may determine alerts/alarms based on any of the information received by mode transition module 132 (e.g., energy/charge level of power source 120, current pump duty cycle, etc.), according to some embodiments.

**Pulse Width Modulation and Duty Cycle Examples**

Referring now to FIG. 3, an illustrative graph 300 of duty cycle resulting from PWM is shown, according to some embodiments. The illustrative graph 300 is shown to include a series 302 actuating between an on state and an off state, according to some embodiments. The y-axis of graph 300 represents the on state and the off state of a controlled equipment (e.g., pump 142), and the x-axis of graph 300 represents time (e.g., time increasing), according to some embodiments. In some embodiments, series 302 is shown being in the on state for time interval 304, and in the off state for time interval 308. In some embodiments, time interval 304 is referred to as a pulse width *PW*. The summation of time interval 304 and time interval 308 is defined as period 306 (*T*), according to some embodiments. In some embodiments, the duty cycle is determined using a duty cycle equation, mathematically represented as $D = \frac{PW}{T} \times 100\%$. In the duty cycle equation shown, *D* is the duty cycle (in terms of %), *PW* is time interval 304, and *T* is period 306, according to some embodiments. In this way, the duty cycle relates the on-time with the off-time, indicating an amount of time the controlled equipment has been in the on-state with respect to period 306. When applied to pumps, duty cycle is a total amount of time the pump is in the on-state over an hour of operation, according to some embodiments. PWM module 136 is configured to modulate the pulse width *PW* (i.e., time interval 304) to achieve various therapy pressure setpoints, according to some embodiments.

**Control Algorithm**

Overview

Referring now to FIGS. 4-6b, control algorithm 400 is described in greater detail, according to some embodiments. Control algorithm 400 includes "increasing" or "decreasing" therapy pressure setpoint $TP_{setpoint}$, according to some embodiments. Since the present disclosure relates to NPWT, "increasing" the therapy pressure setpoint $TP_{setpoint}$ means adjusting the therapy pressure setpoint $TP_{setpoint}$ from a negative pressure value to a more negative pressure value (e.g., from -125 mmHg to -150 mmHg), according to some embodiments. Likewise, "decreasing" the therapy pressure setpoint $TP_{setpoint}$ means adjusting the therapy pressure setpoint $TP_{setpoint}$ from a negative pressure value to a less negative pressure value (e.g., from -150 mmHg to -125 mmHg), according to some embodiments. "Minimum" therapy pressure setpoint $TP_{setpoint}$ means a least negative therapy pressure setpoint $TP_{setpoint}$ value close to zero (e.g., -85 mmHg), while a "maximum" therapy pressure setpoint $TP_{setpoint}$ means a most negative therapy pressure setpoint $TP_{setpoint}$ (e.g., -150 mmHg), according to some embodiments. Similarly, "greater than" in regards to therapy pressure setpoint $TP_{setpoint}$ means more negative (e.g., -150 mmHg is greater than -110 mmHg), and "less than" in regards to therapy pressure setpoint $TP_{setpoint}$ means less negative (e.g., -110 mmHg is less than -150 mmHg), according to some embodiments. In any of FIGS. 4-6b, "increasing" and "decreasing" may be taken to mean increasing or decreasing an absolute value of the therapy pressure setpoint $TP_{setpoint}$, according to some embodiments. Control algorithm 400 also includes increasing or decreasing the therapy pressure setpoint by certain amounts (e.g., $\Delta p$, $\Delta p_{small}$, etc.), according to some embodiments. In some embodiments, the certain amounts are quantities, resulting in the therapy pressure setpoint $TP_{setpoint}$ being linearly increased or decreased. In some embodiments, the amounts are functions of other variables (e.g., time, therapy pressure, pump duty cycle value, energy/charge level, etc.), resulting in the therapy pressure setpoint $TP_{setpoint}$ being increased or decreased non-linearly.

Referring now to FIG. 4, a block diagram of control algorithm 400 of NPWT device 100 is shown, according to some embodiments. In some embodiments, control algorithm 400 illustrates an overview of control algorithms described in greater detail below with reference to FIGS. 5a-6b.

The first step of control algorithm 400 is a startup step 402 and includes starting/initializing NPWT device 100, according to some embodiments. NPWT device 100 may be started by connecting power source 120 to NPWT device 100 and receiving a command from a user to start the NPWT device 100. In some embodiments, the user inputs the command to start NPWT device 100 through user interface 106. In some embodiments, the user inputs the command to start NPWT device 100 through at least one of buttons 104. The startup step 402 also includes setting various initial NPWT parameters (e.g., type of NPWT, duration of NPWT, therapy pressure setpoint of the NPWT, etc.), according to some embodiments. In some embodiments, the user determines the initial NPWT parameters through user interface 106.

After NPWT device 100 has been started and initialized with various NPWT settings, NPWT device 100 enters a standard therapy mode of operation 404, according to some embodiments. In some embodiments, the standard therapy mode of operation 404 corresponds to standard therapy module 122 determining therapy pressure setpoints. In some embodiments, the standard therapy mode of operation 404 includes setting the therapy pressure setpoint of pump 142 to a negative pressure (e.g., -125 mmHg or any other negative pressure determined based on performance requirements), and periodically monitoring the pump duty cycle value of pump 142 to determine if a leak has occurred. If a leak does not occur, NPWT device 100 continues operating in standard therapy mode of operation 404 until the NPWT is completed, according to some embodiments. In some embodiments, standard therapy mode of operation 404 includes calculating the continuous pump duty cycle value as described in greater detail above with reference to FIG. 2. In some embodiments, the continuous pump duty cycle value ensures that the NPWT can be maintained for the prescribed therapy duration.

If the pump duty cycle exceeds a predetermined threshold value, NPWT device 100 transitions out of the standard therapy mode of operation 404 and enters seal assist mode of operation 406, according to some embodiments. In some embodiments, seal assist mode of operation 406 attempts to seal the leak in the vacuum system by increasing the therapy pressure setpoint over a time interval. In some embodiments, pump 142 can provide enough therapy pressure to overcome the leak and operate to perform the NPWT despite the leak, however, if a leak occurs, pump 142 may be required to operate at a higher pump duty cycle value, which may consume energy/charge from power source 120. If the leak is sealed, NPWT device 100 may transition back into standard therapy mode of operation 404, according to some embodiments. Seal assist mode of operation 406 includes ramping up the therapy pressure setpoint within safe limits (e.g., ramping up to -150 mmHg) to attempt to seal the leak, and periodically checking the pump duty cycle to determine if the leak has been sealed (since, as described above, leakage correlates to pump duty cycle), according to some embodiments. If after a predetermined time period, the leak has been sealed (identified by the pump duty cycle returning to an expected value), NPWT device 100 transitions back into standard therapy mode of operation 404, according to some embodiments.

If seal assist mode of operation 406 is unable to seal the leak (e.g., if the leak is too big, or if sealing the leak requires using an undesirable amount of energy/charge from power source 120), NPWT device 100 transitions into therapy pressure optimization mode of operation 408, according to some embodiments. Pressure optimization mode of operation 408 includes determining an efficient pump duty cycle value to provide therapy pressure at a different setpoint, such that the NPWT can be sufficiently performed given the energy/charge remaining in power source 120, according to some embodiments. In some embodiments, pressure optimization mode of operation 408 includes optimizing therapy pressure setpoint based on pump duty cycle value and remaining energy/charge in power source 120. In this way, therapy pressure optimization mode 408 allows NPWT device 100 to continue operating and administering NPWT despite leaks, according to some embodiments. Additionally, therapy pressure optimization mode of operation 408 may take into account remaining energy/charge in power source 120 and determine a therapy pressure setpoint and pump duty cycle which can be maintained for an entirety of the prescribed therapy duration. Advantageously, pressure optimization mode 408 continues the NPWT despite a leak or low energy/charge level of power source 120. In this way, NPWT device 100 is prevented from merely outputting an alarm/alert to the user and shutting down, and provides a more versatile NPWT device which can operate to provide NPWT despite leaks and low energy/charge level. In some embodiments, if the pump duty cycle value exceeds a pump duty cycle threshold value and the energy/charge level of power source 120 is within a first range (e.g., 100%-50%), the therapy pressure setpoint is incrementally lowered (e.g., reduced by 10 mmHg every 30 minutes) until the pump duty cycle value is below the pump duty cycle threshold value. This works on the principle that by lowering the therapy pressure, leak rate also lowers proportionally, according to some embodiments. When the energy/charge level of power source 120 is within the first range (e.g., 100%-50%), NPWT device 100 has time to perform corrective measures to ensure therapy duration is maintained. If after an initial reduction in therapy pressure (e.g., an initial reduction of 10 mmHg), the pump duty cycle value is significantly lower than the pump duty cycle threshold value (e.g., 10% lower, 5% lower, etc.), therapy pressure may be gradually increased in smaller increments (e.g., 1 mmHg every 30 minutes) to maximize therapy pressure and balance against pump duty cycle, according to some embodiments.

If the energy/charge level of power source 120 is within a second range (e.g., 50%-20%), therapy pressure setpoint may be incrementally reduced in larger increments or in a shorter time duration (e.g., reduced 20 mmHg every 30 minutes, or reduced 10 mmHg every 15 minutes, etc.) to speed up the optimization process of therapy pressure optimization mode of operation 408, according to some embodiments. The increased rate at which the therapy pressure setpoint is reduced may result in larger deviations in the optimization process which may require additional steps of incrementally increasing the therapy pressure to fully optimize the therapy pressure setpoint with respect to the pump duty cycle value (e.g., leak rate), according to some embodiments.

If the energy/charge level of power source 120 is less than a lower bounds of the second range (e.g., below 20%), therapy pressure setpoint is immediately reduced to a minimum allowable therapy pressure setpoint (e.g., -75 mmHg) to conserve energy/charge remaining in power source 120 as much as possible and to attempt to maintain at least two hours of therapy duration to provide the user with sufficient time to arrange for the replacement of the power source 120 or to arrange for a new device to be fitted, according to some embodiments. If it is determined after the NPWT device 100 is at the minimum allowable therapy pressure setpoint that the NPWT device 100 can provide NPWT at a higher therapy pressure setpoint for two hours of therapy, the therapy pressure setpoint is incrementally increased until a therapy pressure setpoint is reached which can still be maintained for at least two hours.

If NPWT device 100 cannot provide NPWT at the minimum allowable therapy pressure setpoint and/or if energy/charge level of power source 120 decreases below a minimum threshold value, an alarm/alert is provided to the user through user interface 106 and NPWT device 100 transitions into preservation mode of operation 410, according to some embodiments. Preservation mode of operation 410 includes lowering the therapy pressure setpoint to a new minimum value, working on the basis that some negative pressure is better than none. This principle works with absorbent dressing due to no head of fluid, according to some embodiments. For devices which exudate canisters, the new minimum value of the therapy pressure setpoint is determined based on tube length, according to some embodiments.

## Detailed Control Algorithm

Referring now to FIGS. 5a-5c, a flowchart illustrating the control algorithm 400 described above with reference to FIG. 4 is shown in greater detail, according to some embodiments. Control algorithm 400 is shown to include standard therapy mode of operation 501, seal assist mode of operation 503, pressure optimization mode of operation 505, and preservation mode of operation 507, according to some embodiments. In some embodiments, standard therapy mode of operation 501 is standard therapy mode of operation 404, seal assist mode of operation 503 is seal assist mode of operation 406, therapy pressure optimization mode of operation 505 is therapy pressure optimization mode of operation 408, and preservation mode of operation 507 is preservation mode of operation 410, as described above with reference to FIG. 4. In some embodiments, standard therapy mode of operation 501/404 is performed by standard therapy module 122, seal assist mode of operation 503/406 is performed by seal assist module 124, pressure optimization mode of operation 505/408 is performed by pressure optimization module 126, and preservation mode of operation 507/410 is performed by preservation mode module 128 of controller 110. In some embodiments, any of the methods or logic for transitioning between any of the various modes of operation is performed by mode transition module 132 of controller 110.

The first step 502 of control algorithm 400 includes starting and initializing NPWT device 100, according to some embodiments. In some embodiments, step 502 as shown in FIG. 5a is the same as step 402 shown in FIG. 4 and described in greater detail above with reference to FIG. 4. After NPWT device 100 has been started and initialized, NPWT device 100 enters standard therapy mode of operation 501, according to some embodiments.

The standard therapy mode of operation 501 first sets the therapy pressure setpoint, $TP_{setpoint}$, to an initial therapy pressure, $p_i$, (step 504) according to some embodiments. In some embodiments, the initial therapy pressure, $p_i$, is -125 mmHg. In some embodiments, the initial therapy pressure $p_i$ is an initial therapy therapy pressure. Standard therapy mode of operation 501 next compares the pump duty cycle value, *PD*, to a pump duty cycle threshold value, $X$, (step 506) according to some embodiments. In some embodiments, if the pump duty cycle value $PD$ is less than or equal to the pump duty cycle threshold value $X$, NPWT device 100 continues operating according to standard therapy mode of operation 501 (i.e., returns to step 504).

If, however, the pump duty cycle value $PD$ is greater than the pump duty cycle threshold value $X$, this indicates that a leak has occurred and NPWT device 100 transitions into seal assist mode of operation 503 to attempt to seal the leak, according to some embodiments. Seal assist mode of operation 503 first compares therapy pressure setpoint $TP_{setpoint}$ to a new therapy pressure, $p_{new}$ (step 508), according to some embodiments. In some embodiments, the new therapy pressure $p_{new}$ is greater than the initial therapy pressure $p_i$. In some embodiments, the new therapy pressure $p_{new}$ is -150 mmHg. If the therapy pressure setpoint $TP_{setpoint}$ is not equal to the new therapy pressure $p_{new}$, the therapy pressure setpoint $TP_{setpoint}$ is set equal to the new therapy pressure $p_{new}$ (step 510), according to some embodiments. After the therapy pressure setpoint $TP_{setpoint}$ is set equal to the new therapy pressure $p_{new}$, a timer is started (step 512) for a predetermined amount of time t, according to some embodiments. In some embodiments, time t is 2.5 minutes. The pump duty cycle value $PD$ is periodically compared to pump duty cycle threshold value $X$ (e.g., periodically at an end of a time step such as every 1 second), according to some embodiments. If at any time the pump duty cycle value $PD$ falls below or is equal to the pump duty cycle threshold value $X$, the NPWT device 100 is transitioned out of the seal assist mode of operation 503 and into the standard therapy mode of operation 501 (since the pump duty cycle value returning to an acceptable value indicates that the leak has been sealed) according to some embodiments. If, however, the pump duty cycle value $PD$ does not fall below the pump duty cycle threshold value $X$ (step 506) and the timer is not greater than or equal to time t (step 514), seal assist mode of operation 503 continues to periodically check both the pump duty cycle value $PD$ (step 506) and the therapy pressure setpoint $TP_{setpoint}$ (step 508), according to some embodiments. Once the timer reaches time t, the therapy pressure setpoint $TP_{setpoint}$ is set to the initial therapy pressure $p_i$ (step 514 and step 516), according to some embodiments. In some embodiments, NPWT device 100 is then allowed to operate at the initial therapy pressure for a predetermined amount of time. After the therapy pressure setpoint $TP_{setpoint}$ is set to the initial therapy pressure $p_i$, the pump duty cycle value $PD$ is again compared to the pump cycle threshold value $X$ (step 518), according to some embodiments. If the pump duty cycle value $PD$ is less than or equal to the pump duty cycle threshold value $X$, NPWT device 100 is transitioned from the seal assist mode of operation 503 into the standard therapy mode of operation 501 (since pump duty cycle value returning to an acceptable value indicates that the leak has been sealed), according to some embodiments. If the pump duty cycle value $PD$ is greater than the pump duty cycle threshold value $X$, NPWT device 100 is transitioned from seal assist mode of operation 503 into pressure optimization mode of operation 505, since this indicates that the leak has not and/or cannot be sealed with seal assist mode of operation 503, according to some embodiments. In some embodiments, an actual therapy pressure $TP_{actual}$ is measured at the end of time $t$. If the actual therapy pressure $TP_{actual}$ does not equal $p_{new}$ at the end of time $t$, NPWT device 100 is transitioned from seal assist mode of operation 503 into pressure optimization mode of operation 505, according to some embodiments.

Referring now to FIG. 5b, pressure optimization mode of operation 505 of control algorithm 400 is shown, according to some embodiments. Pressure optimization mode of operation 505 first reduces the therapy pressure setpoint value $TP_{setpoint}$ by a determined amount $\Delta p$ (step 520), according to some embodiments. In some embodiments, the value of $\Delta p$ is determined based on energy/charge level of power source 120, according to some embodiments. For example, if the energy/charge level of power source 120 is between a first range (e.g., 100%-50%), $\Delta p$ may equal 10 mmHg, according to some embodiments. If the energy/charge level of power source 120 is between a second range (e.g., 50%-20%), $\Delta p$ may equal 15 mmHg, according to some embodiments. In some embodiments, the value of $\Delta p$ is inversely proportional to the energy/charge level of power source 120, such that lower energy/charge levels of power source 120 correspond to a higher value of $\Delta p$. In this way, pressure optimization mode of operation 505 reduces $TP_{setpoint}$ in larger increments if the energy/charge level of power source 120 is low, according to some embodiments. Pressure optimization mode of operation 505 next checks if the pump duty cycle value $PD$ is less than or equal to the pump duty cycle threshold value $X$ (step 522), according to some embodiments. If the pump duty cycle value $PD$ is greater than the pump duty cycle threshold value $X$ (step 522) and the therapy pressure setpoint $TP_{setpoint}$ is greater than a minimum therapy pressure value $p_{min}$ (step 524), pressure optimization mode of operation 505 continues reducing the setpoint therapy pressure $TP_{setpoint}$ by $\Delta p$, according to some embodiments. In some embodiments, the minimum therapy pressure is -75 mmHg. Pressure optimization mode of operation 505 continues reducing the therapy pressure setpoint $TP_{setpoint}$ by $\Delta p$ until either the pump duty cycle value $PD$ is less than or equal to the pump duty cycle threshold value $X$ (step 522) or until the therapy pressure setpoint $TP_{setpoint}$ is less than or equal to the minimum therapy pressure $p_{min}$ (step 524), according to some embodiments. In some embodiments, the therapy pressure setpoint $TP_{setpoint}$ is repeatedly reduced by $\Delta p$ at an end of a time step having a duration $\Delta t$. In some embodiments, the time step duration $\Delta t$ is determined similarly to the determination of the value of $\Delta p$. For example, $\Delta t$ may be determined based on energy/charge level of power source 120, according to some embodiments. For example, if the energy/charge level of power source 120 is between the first range (e.g., 100%-50%), $\Delta t$ may equal 30 minutes, according to some embodiments. If the energy/charge level of power source 120 is between a second range (e.g., 50%-20%), $\Delta t$ may equal 15 minutes, according to some embodiments. In some embodiments, the value of $\Delta t$ is proportional to the energy/charge level of power source 120, such that lower energy/charge levels of power source 120 correspond to a lower value of $\Delta t$. In this way, pressure optimization mode of operation 505 reduces $TP_{setpoint}$ more often (i.e., at lower $\Delta t$ values) if the energy/charge level of power source 120 is low, according to some embodiments.

If at any point in time while pressure optimization mode of operation 505 is reducing $TP_{setpoint}$, the pump duty cycle value *PD* is less than or equal to the pump duty cycle threshold value *X* (step 522), pressure optimization mode of operation 505 maintains the current $TP_{setpoint}$ value (step 526), according to some embodiments. Pressure optimization mode of operation 505 then determines if the pump duty cycle value *PD* is less than or equal to the pump duty cycle threshold value *X* (step 526), according to some embodiments. If the pump duty cycle value *PD* is less than or equal to the pump duty cycle threshold value *X* (step 528), NPWT device 100 is transitioned from pressure optimization mode of operation 505 into standard therapy mode of operation 501, according to some embodiments. If the pump duty cycle value *PD* is still greater than the pump duty cycle threshold value *X* (step 528), pressure optimization mode of operation 505 returns to reducing the therapy pressure setpoint $TP_{setpoint}$ (steps 520-524), according to some embodiments.

If, while pressure optimization mode of operation 505 is reducing the therapy pressure setpoint $TP_{setpoint}$, the therapy pressure setpoint $TP_{setpoint}$ falls below the minimum therapy pressure value $p_{min}$ (step 524), NPWT device 100 is transitioned from pressure optimization mode of operation 505 to preservation mode of operation 507, according to some embodiments. The goal of pressure optimization mode of operation 505 is to determine a therapy pressure setpoint $TP_{setpoint}$ which can be maintained at an acceptable pump duty cycle value, according to some embodiments. If however, pressure optimization mode of operation 505 causes the therapy pressure setpoint $TP_{setpoint}$ to fall below the minimum therapy pressure $p_{min}$, NPWT device 100 is transitioned out of pressure optimization mode of operation 505 into preservation mode of operation 507, according to some embodiments.

Additionally, if the energy/charge level of power source 120 is less than a lower bounds of the second range (e.g., less than 20%), pressure optimization mode of operation 505 immediately reduces the therapy pressure setpoint $TP_{setpoint}$ to the minimum therapy pressure $p_{min}$, according to some embodiments.

Referring now to FIG. 5c, preservation mode of operation 507 of control algorithm 400 is shown, according to some embodiments. When NPWT device 100 is transitioned into preservation mode of operation 507, a leak alert is provided to the user through user interface 106 (step 530), according to some embodiments. In some embodiments, the leak alert is at least one of an auditory alert and a visual alert. Preservation mode of operation 507 next sets the therapy pressure setpoint $TP_{setpoint}$ equal to the minimum therapy pressure $p_{min}$ (step 532), according to some embodiments. Preservation mode of operation 507 next checks if the pump duty cycle value *PD* is less than or equal to the pump duty cycle threshold value *X* (step 534), according to some embodiments. If the pump duty cycle value *PD* is less than or equal to the pump duty cycle threshold value *X* (step 534), preservation mode of operation 507 maintains the current therapy pressure setpoint $TP_{setpoint}$, according to some embodiments. In some embodiments, preservation mode of operation 507 maintains the current therapy pressure setpoint $TP_{setpoint}$ for a predetermined amount of time (step 542). If, after the predetermined amount of time, the pump duty cycle value *PD* is less than or equal to the pump duty cycle threshold value *X* (step 544) and the current setpoint therapy pressure $TP_{setpoint}$ is maintained, NPWT device 100 is transitioned from preservation mode of operation 507 into standard therapy mode of operation 501, according to some embodiments. In some embodiments, NPWT device 100 is only transitioned from preservation mode of operation 507 into standard therapy mode of operation 501 if the energy/charge level of power source 120 exceeds a predetermined threshold value (e.g., is above 20%, is above 50%, etc.).

If the pump duty cycle value *PD* is greater than the pump duty cycle threshold value *X* (step 534), and the setpoint therapy pressure $TP_{setpoint}$ does not equal a new minimum therapy pressure $p_{min,new}$ (step 536), the therapy pressure setpoint $TP_{setpoint}$ is reduced by amount $\Delta p$, according to some embodiments. In some embodiments, the new minimum therapy pressure $p_{min,new}$ equals -25 mmHg. In some embodiments $\Delta p$ is 10 mmHg. Preservation mode of operation 507 repeatedly reduces the therapy pressure setpoint $TP_{setpoint}$ by the new amount $\Delta p_{\text{new}}$ until either the pump duty cycle value *PD* is less than or equal to the pump duty cycle threshold value *X* or until the therapy pressure setpoint $TP_{setpoint}$ equals the new minimum therapy pressure $p_{min,new}$, according to some embodiments. Once the therapy pressure setpoint $TP_{setpoint}$ substantially equals the new minimum therapy pressure $p_{min,new}$ (step 536), intermittent therapy is applied at the new minimum therapy pressure $p_{min,new}$ (step 540), according to some embodiments. In some embodiments, the intermittent therapy is applied at a pump duty cycle value of 50%. For example, the intermittent therapy may be repeatedly applied at the therapy pressure setpoint $TP_{setpoint}$ equaling the new minimum therapy pressure $p_{min,new}$ for five minutes on, and five minutes off, according to some embodiments.

If at any point in time when preservation mode of operation 507 is reducing the therapy pressure setpoint $TP_{setpoint}$ by $\Delta p$ (steps 534-538), the pump duty cycle value *PD* is less than the pump duty cycle threshold value *X* (step 534), preservation mode of operation 507 maintains the current therapy pressure setpoint $TP_{setpoint}$ (step 542), according to some embodiments. In some embodiments, preservation mode of operation 507 maintains the current therapy pressure setpoint $TP_{setpoint}$ for a predetermined amount of time (step 542). If, after the predetermined amount of time, the pump duty cycle value *PD* is less than or equal to the pump duty cycle threshold value *X* (step 544) and the current therapy pressure setpoint $TP_{setpoint}$ is maintained, NPWT device 100 is transitioned from preservation mode of operation 507 into standard therapy mode of operation 501, according to some embodiments. In some embodiments, NPWT device 100 is only transitioned from preservation mode of operation 507 into standard therapy mode of operation 501 if the energy/charge level of power source 120 exceeds a predetermined threshold value (e.g., is above 20%, is above 50%, etc.). If after the predetermined amount of time, however, the pump duty cycle value *PD* is greater than the pump duty cycle threshold value *X* (step 544), preservation mode of operation 507 resumes reducing the therapy pressure setpoint $TP_{setpoint}$ by $\Delta p$ (steps 543-538), according to some embodiments.

Alternative Pressure Optimization Mode and Preservation Mode

Referring now to FIGS. 6a-6b, alternative pressure optimization mode of operation 505 and preservation mode of operation 507 of control algorithm 400 are shown, according to some embodiments. Pressure optimization mode of operation 505 as shown in FIG. 6a includes steps 602-610, according to some embodiments.

Referring to FIG. 6a, pressure optimization mode of operation 505 first reduces the therapy pressure setpoint value $TP_{setpoint}$ by a determined amount $\Delta p$ (step 520), according to some embodiments. In some embodiments, the value of $\Delta p$ is determined based on energy/charge level of power source 120, according to some embodiments. For example, if the energy/charge level of power source 120 is between a first range (e.g., 100%-50%), $\Delta p$ may equal 10 mmHg, according to some embodiments. If the energy/charge level of power source 120 is between a second range (e.g., 50%-20%), $\Delta p$ may equal 15 mmHg, according to some embodiments. In some embodiments, the value of $\Delta p$ is inversely proportional to the energy/charge level of power source 120, such that lower energy/charge levels of power source 120 correspond to a higher value of $\Delta p$. In this way, pressure optimization mode of operation 505 reduces $TP_{setpoint}$ in larger increments if the energy/charge level of power source 120 is low, according to some embodiments. Pressure optimization mode of operation 505 next checks if the pump duty cycle value *PD* is greater than or equal to the pump duty cycle threshold value *X* (step 602), according to some embodiments. If the pump duty cycle value *PD* is greater than the pump duty cycle threshold value *X* (step 522) and the therapy pressure setpoint $TP_{setpoint}$ is greater than a minimum therapy pressure value $p_{min}$ (step 524), pressure optimization mode of operation 505 continues reducing the therapy pressure setpoint $TP_{setpoint}$ by $\Delta p$, according to some embodiments. In some embodiments, the minimum therapy pressure is -75 mmHg. In some embodiments, the minimum therapy pressure is -85 mmHg. Pressure optimization mode of operation 505 continues reducing the therapy pressure setpoint $TP_{setpoint}$ by $\Delta p$ until either the pump duty cycle value *PD* is less than the pump duty cycle threshold value *X* (step 602) or until the therapy pressure setpoint $TP_{setpoint}$ is less than the minimum therapy pressure $p_{min}$ (step 524), according to some embodiments.

If the pump duty cycle value *PD* is less than the pump duty cycle threshold value *X* (step 602), pressure optimization mode of operation 505 checks if the pump duty cycle value *PD* is less than or equal to a second pump duty cycle threshold value *Y*, according to some embodiments. If the pump duty cycle value *PD* is less than the second pump duty cycle threshold value Y, pressure optimization mode of operation 505 increases the setpoint therapy pressure setpoint $TP_{setpoint}$ by $\Delta p_{small}$ (step 606), according to some embodiments. In some embodiments, $\Delta p_{small}$ is 1 mmHg. Next, the therapy pressure setpoint $TP_{setpoint}$ is compared to the initial therapy pressure $p_i$ (step 610), according to some embodiments. If the therapy pressure setpoint $TP_{setpoint}$ does not equal the initial therapy pressure $p_i$, step 602, step 604, and step 606 are repeated (provided that the pump duty cycle value *PD* meets the criteria of step 602 and step 604). In this way, the therapy pressure setpoint $TP_{setpoint}$ is repeatedly increased by $\Delta P_{small}$ (step 606), provided that the pump duty cycle value *PD* meets the criteria of step 602 and step 604, according to some embodiments. If the pump duty cycle value *PD* meets the criteria of step 602 and step 604, and the therapy pressure setpoint $TP_{setpoint}$ is increased until it equals the initial therapy pressure $p_i$, NPWT device 100 is transitioned out of pressure optimization mode of operation 505 and into standard therapy mode of operation 501, according to some embodiments. In some embodiments, NPWT device 100 is only transitioned out of pressure optimization mode of operation 305 and into standard therapy mode of operation 501 if energy/charge level of power source 120 exceeds a predetermined value (e.g., is greater than 50%, is greater than 70%, etc.). In this way, NPWT device 100 cannot be transitioned back into standard therapy mode of operation 501 if energy/charge level of power source 120 is not sufficient to provide NPWT according to standard therapy mode of operation 501 for the prescribed therapy duration.

Referring now to FIG. 6b, preservation mode of operation 507 performs steps 530-542 as described above in greater detail with reference to FIG. 5c, according to some embodiments. However, if the pump duty cycle value *PD* is less than the pump duty cycle threshold value *X* (step 534), preservation mode of operation then determines if the pump duty cycle value *PD* is less than or equal to the second pump duty cycle threshold value *Y* (step 612), according to some embodiments. If the pump duty cycle value *PD* is greater than the second pump duty cycle threshold value *Y*, preservation mode of operation 507 performs step 542, according to some embodiments. If, however, the pump duty cycle value *PD* is less than or equal to the second pump duty cycle threshold value *Y* (step 612), the therapy pressure setpoint $TP_{setpoint}$ is increased by $\Delta p_{small}$ (step 614), according to some embodiments. In some embodiments, $\Delta p_{small}$ is 1 mmHg. After the therapy pressure setpoint $TP_{setpoint}$ is increased by $\Delta p_{small}$, the therapy pressure setpoint $TP_{setpoint}$ is compared to $p_i$ (step 610), according to some embodiments. If the therapy pressure setpoint $TP_{setpoint}$ is equal to $p_i$, NPWT device 100 is transitioned back into standard therapy mode of operation 501, according to some embodiments. If the therapy pressure setpoint $TP_{setpoint}$ is not equal to $p_i$, preservation mode of operation 507 returns to step 534, according to some embodiments. In this way, preservation mode of operation 507 may gradually increase the therapy pressure setpoint $TP_{setpoint}$ to attempt and provide as much negative pressure as possible.

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the disclosure as recited in the appended claims.

It should be noted that the term "exemplary" and variations thereof, as used herein to describe various embodiments, are intended to indicate that such embodiments are possible examples, representations, or illustrations of possible embodiments (and such terms are not intended to connote that such embodiments are necessarily extraordinary or superlative examples).

The hardware and data processing components used to implement the various processes, operations, illustrative logics, logical blocks, modules and circuits described in connection with the embodiments disclosed herein may be implemented or performed with a general purpose single- or multi-chip processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein. A general purpose processor may be a microprocessor, or, any conventional processor, controller, microcontroller, or state machine. A processor also may be implemented as a combination of computing devices, such as a combination of a DSP and a microprocessor, a plurality of microprocessors, one or more microprocessors in conjunction with a DSP core, or any other such configuration. In some embodiments, particular processes and methods may be performed by circuitry that is specific to a given function. The memory (e.g., memory, memory unit, storage device) may include one or more devices (e.g., RAM, ROM, Flash memory, hard disk storage) for storing data and/or computer code for completing or facilitating the various processes, layers and modules described in the present disclosure. The memory may be or include volatile memory or non-volatile memory, and may include database components, object code components, script components, or any other type of information structure for supporting the various activities and information structures described in the present disclosure. According to an exemplary embodiment, the memory is communicably connected to the processor via a processing circuit and includes computer code for executing (e.g., by the processing circuit or the processor) the one or more processes described herein.

The present disclosure contemplates methods, systems and program products on any machine-readable media for accomplishing various operations. The embodiments of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. Combinations of the above are also included within the scope of machine-readable media. Machine-executable instructions include, for example, instructions and data which cause a general purpose computer, special purpose computer, or special purpose processing machines to perform a certain function or group of functions.

## Claims

1. A negative pressure wound therapy, NPWT, device (100) configured to perform NPWT, the NPWT device (100) comprising:

   a battery (120) configured to supply the NPWT device (100) with power;
   a pump (142) configured to receive power from the battery (120) and to produce a vacuum at a setpoint pressure to perform the NPWT;
   a user interface (106) configured to provide alerts to a user;
   a controller (110) configured to receive power from the battery (120) and to adjust the setpoint pressure of the pump and further configured to:

      operate the NPWT device (100) in a standard therapy mode, wherein the standard therapy mode comprises operating the pump (142) at a first setpoint vacuum pressure and periodically comparing a determined duty cycle value of the pump (142) to a predetermined duty cycle threshold value;
      operate the NPWT device (100) in a seal assist therapy mode, wherein the seal assist therapy mode comprises operating the pump (142) at a second setpoint vacuum pressure, wherein the second setpoint vacuum pressure is greater than the first setpoint vacuum pressure;
      **characterized in that** the controller (110) is configured to operate the NPWT device (100) in a pressure optimization mode, wherein the pressure optimization mode comprises:

         determining an initial pump duty cycle value and an initial battery capacity;
         reducing the setpoint pressure of the vacuum by a determined amount at an end of a timestep;
         determining a second pump duty cycle value at the end of the timestep;
         monitoring an actual vacuum pressure produced by the pump (142) at the end of the timestep;
         repeating the steps of reducing the setpoint pressure, calculating the second pump duty cycle value, and monitoring the actual vacuum pressure of the pump (142) until the second pump duty cycle value is less than the predetermined duty cycle threshold value; and wherein the determined amount and the timestep are determined based on at least one of the initial pump duty cycle value and the initial battery capacity; and

      operate the NPWT device (100) in a preservation mode of operation, wherein the preservation mode of operation comprises reducing the setpoint vacuum pressure to an absolute minimum pressure.

2. The NPWT device of Claim 1, wherein the controller (110) is further configured to cause the user interface (106) to provide an alert, wherein the alert indicates at least one of a leak event and a low battery capacity, wherein the alert optionally comprises at least one of a visual alert and an auditory alert.

3. The NPWT device of Claim 1, wherein the controller (110) is further configured to determine a continuous pump duty value, wherein the continuous pump duty value ensures that NPWT can be maintained for a therapy time duration, wherein the continuous pump duty value is optionally determined based on at least one of properties of the pump (142), battery capacity of the battery (120), and the therapy time duration.

4. The NPWT device of Claim 1, wherein the controller (110) is further configured to transition the NPWT device (100) from the standard therapy mode into the seal assist therapy mode in response to the determined duty cycle value exceeding the predetermined duty cycle threshold value.

5. The NPWT device of Claim 1, wherein the standard therapy mode further comprises periodically actuating the pump (142) between an on state and an off state to produce the first setpoint vacuum pressure.

6. The NPWT device of Claim 1, wherein the seal assist therapy mode further comprises repeatedly increasing the setpoint pressure of the pump (142) until the pump achieves the second vacuum pressure.

7. The NPWT device of Claim 6, wherein the seal assist therapy mode further comprises adjusting the pump (142) to operate at the first vacuum pressure for a predetermined time period in response to the pump achieving the second setpoint vacuum pressure.

8. The NPWT device of Claim 7, wherein the seal assist therapy mode further comprises calculating the pump duty cycle value of the pump (142) and monitoring the actual vacuum pressure of the pump at an end of the predetermined time period.

9. The NPWT device of Claim 8, wherein the controller (110) is configured to transition the NPWT device (100) from the seal assist therapy mode into the standard therapy mode in response to the calculated pump duty cycle value of the pump (142) at the end of the predetermined time period being less than the predetermined duty cycle threshold value.

**10.** The NPWT device of Claim 8, wherein the controller (110) is configured to transition the NPWT device (100) from the seal assist therapy mode into the pressure optimization mode in response to at least one of:

the calculated pump duty cycle value of the pump (142) at the end of the predetermined time period being greater than the predetermined duty cycle threshold value; and
the monitored actual vacuum pressure of the pump (142) at the end of the predetermined time period being less than the second setpoint vacuum pressure.

**11.** The NPWT device of Claim 1, wherein the pressure optimization mode further comprises:

increasing the setpoint vacuum pressure by a predetermined amount;
calculating the pump duty cycle; and
repeating the steps of increasing the setpoint vacuum pressure and calculating the pump duty cycle until the calculated pump duty cycle is substantially equal to the predetermined duty cycle threshold value.

**12.** The NPWT device of Claim 1, wherein the pressure optimization mode further comprises causing the user interface (106) to display a leak alert in response to the monitored actual vacuum pressure falling below a minimum allowable vacuum pressure.

**13.** The NPWT device of Claim 1, wherein the pressure optimization mode further comprises maintaining a current vacuum pressure for a pressure maintenance time duration in response to the second pump duty cycle value being less than the predetermined duty cycle threshold value, wherein the controller (110) is optionally configured to transition the NPWT device (100) from the pressure optimization mode into the standard therapy mode in response to the pump duty cycle value at an end of the pressure maintenance time duration being less than the predetermined duty cycle threshold value.

**14.** The NPWT device of Claim 1, wherein the controller (110) is configured to:

determine a difference between the monitored actual vacuum pressure and a minimum threshold vacuum pressure; and
transition the NPWT device (100) from the pressure optimization mode of operation to the preservation mode of operation based on the determined difference between the monitored actual vacuum pressure and the minimum threshold vacuum pressure.

**15.** The NPWT device of Claim 1, wherein the preservation mode of operation further comprises repeatedly reducing the setpoint vacuum pressure until the setpoint vacuum pressure is substantially equal to the absolute minimum pressure, wherein the preservation mode of operation optionally comprises operating the pump (142) to intermittently produce the absolute minimum pressure.

## Patentansprüche

**1.** Eine Unterdruck-Wundtherapievorrichtung, NPWT-Vorrichtung, (100), die konfiguriert ist, um NPWT durchzuführen, die NPWT-Vorrichtung (100) aufweisend:

eine Batterie (120), die konfiguriert ist, um die NPWT-Vorrichtung (100) mit Strom zu versorgen;
eine Pumpe (142), die konfiguriert ist, um Strom von der Batterie (120) zu empfangen und um ein Vakuum bei einem Solldruck zu erzeugen, um die NPWT durchzuführen;
eine Benutzerschnittstelle (106), die konfiguriert ist, um einem Benutzer Warnungen bereitzustellen;
eine Steuerung (110), die konfiguriert ist, um Strom von der Batterie (120) zu empfangen und um den Solldruck der Pumpe einzustellen, und ferner konfiguriert ist zum:

Betreiben der NPWT-Vorrichtung (100) in einem Standardtherapiemodus, wobei der Standardtherapiemodus das Betreiben der Pumpe (142) bei einem ersten Sollvakuumdruck und ein periodisches Vergleichen eines bestimmten Arbeitszykluswerts der Pumpe (142) mit einem zuvor bestimmten Arbeitszyklusschwellenwert aufweist;
Betreiben der NPWT-Vorrichtung (100) in einem Abdichtungsunterstützungstherapiemodus, wobei der Abdichtungsunterstützungstherapiemodus das Betreiben der Pumpe (142) bei einem zweiten Sollvakuumdruck aufweist, wobei der zweite Sollvakuumdruck größer als der erste Sollvakuumdruck ist;
**dadurch gekennzeichnet, dass** die Steuerung (110) konfiguriert ist, um die NPWT-Vorrichtung (100) in einem Druckoptimierungsmodus zu betreiben, wobei der Druckoptimierungsmodus aufweist:

Bestimmen eines anfänglichen Pumpenarbeitszykluswerts und einer anfänglichen Batteriekapazität;
Reduzieren des Solldrucks des Vakuums um einen bestimmten Betrag an einem Ende eines Zeitschritts;
Bestimmen eines zweiten Pumpenarbeitszykluswerts an dem Ende des Zeitschritts;
Überwachen eines tatsächlichen Vakuumdrucks, der durch die Pumpe (142) an dem Ende des Zeitschritts erzeugt wird;
Wiederholen der Schritte des Reduzierens des Solldrucks, eines Berechnens des zweiten Pumpenarbeitszykluswerts und des Überwachens des tatsächlichen Vakuumdrucks der Pumpe (142), bis der zweite Pumpenarbeitszykluswert kleiner als der zuvor bestimmte Arbeitszyklusschwellenwert ist; und wobei die bestimmte Menge und der Zeitschritt basierend auf mindestens einem des anfänglichen Pumpenarbeitszykluswerts und der anfänglichen Batteriekapazität bestimmt werden; und
Betreiben der NPWT-Vorrichtung (100) in einem Erhaltungsbetriebsmodus, wobei der Erhaltungsbetriebsmodus das Reduzieren des Sollvakuumdrucks auf einen absoluten Mindestdruck aufweist.

2. Die NPWT-Vorrichtung nach Anspruch 1, wobei die Steuerung (110) ferner konfiguriert ist, um die Benutzerschnittstelle (106) zu veranlassen, eine Warnung bereitzustellen, wobei die Warnung mindestens eines von einem Leckereignis und einer niedrigen Batteriekapazität angibt, wobei die Warnung optional mindestens eine von einer visuellen Warnung und einer akustischen Warnung aufweist.

3. Die NPWT-Vorrichtung nach Anspruch 1, wobei die Steuerung (110) ferner konfiguriert ist, um einen kontinuierlichen Pumpenarbeitswert zu bestimmen, wobei der kontinuierliche Pumpenarbeitswert sicherstellt, dass NPWT für eine Therapiezeitdauer aufrechterhalten werden kann, wobei der kontinuierliche Pumpenarbeitswert optional basierend auf mindestens einer von Eigenschaften der Pumpe (142), Batteriekapazität der Batterie (120) und der Therapiezeitdauer bestimmt wird.

4. Die NPWT-Vorrichtung nach Anspruch 1, wobei die Steuerung (110) ferner konfiguriert ist, um die NPWT-Vorrichtung (100) von dem Standardtherapiemodus in den Abdichtungsunterstützungstherapiemodus zu überführen, als Reaktion darauf, dass der bestimmte Arbeitszykluswert den zuvor bestimmten Arbeitszyklusschwellenwert überschreitet.

5. Die NPWT-Vorrichtung nach Anspruch 1, wobei der Standardtherapiemodus ferner ein periodisches Betätigen der Pumpe (142) zwischen einem eingeschalteten Zustand und einem ausgeschalteten Zustand aufweist, um den ersten Sollvakuumdruck zu erzeugen.

6. Die NPWT-Vorrichtung nach Anspruch 1, wobei der Abdichtungsunterstützungstherapiemodus ferner ein wiederholtes Erhöhen des Solldrucks der Pumpe (142) aufweist, bis die Pumpe den zweiten Vakuumdruck erreicht.

7. Die NPWT-Vorrichtung nach Anspruch 6, wobei der Abdichtungsunterstützungstherapiemodus ferner ein Einstellen der Pumpe (142) aufweist, um bei dem ersten Vakuumdruck für einen zuvor bestimmten Zeitraum in Betrieb zu sein, als Reaktion darauf, dass die Pumpe den zweiten Sollvakuumdruck erreicht.

8. Die NPWT-Vorrichtung nach Anspruch 7, wobei der Abdichtungsunterstützungstherapiemodus ferner das Berechnen des Pumpenarbeitszykluswerts der Pumpe (142) und das Überwachen des tatsächlichen Vakuumdrucks der Pumpe an einem Ende des zuvor bestimmten Zeitraums aufweist.

9. Die NPWT-Vorrichtung nach Anspruch 8, wobei die Steuerung (110) konfiguriert ist, um die NPWT-Vorrichtung (100) von dem Abdichtungsunterstützungstherapiemodus in den Standardtherapiemodus zu überführen, als Reaktion darauf, dass der berechnete Pumpenarbeitszykluswert der Pumpe (142) an dem Ende des zuvor bestimmten Zeitraums kleiner als der zuvor bestimmte Arbeitszyklusschwellenwert ist.

10. Die NPWT-Vorrichtung nach Anspruch 8, wobei die Steuerung (110) konfiguriert ist, um die NPWT-Vorrichtung (100) von dem Abdichtungsunterstützungstherapiemodus in den Druckoptimierungsmodus zu überführen, als Reaktion auf mindestens eines von:

der berechnete Pumpenarbeitszykluswert der Pumpe (142) an dem Ende des zuvor bestimmten Zeitraums ist größer als der zuvor bestimmte Arbeitszyklusschwellenwert; und
der überwachte tatsächliche Vakuumdruck der Pumpe (142) an dem Ende des zuvor bestimmten Zeitraums ist kleiner als der zweite Sollvakuumdruck.

**11.** Die NPWT-Vorrichtung nach Anspruch 1, wobei der Druckoptimierungsmodus ferner aufweist:

Erhöhen des Sollvakuumdrucks um einen zuvor bestimmten Betrag;
Berechnen des Pumpenarbeitszyklus; und
Wiederholen der Schritte des Erhöhens des Sollvakuumdrucks und des Berechnens des Pumpenarbeitszyklus, bis der berechnete Pumpenarbeitszyklus im Wesentlichen gleich dem zuvor bestimmten Arbeitszyklusschwellenwert ist.

**12.** Die NPWT-Vorrichtung nach Anspruch 1, wobei der Druckoptimierungsmodus ferner ein Veranlassen der Benutzerschnittstelle (106) aufweist, eine Leckwarnung als Reaktion darauf anzuzeigen, dass der überwachte tatsächliche Vakuumdruck unter einen zulässigen Mindestvakuumdruck fällt.

**13.** Die NPWT-Vorrichtung nach Anspruch 1, wobei der Druckoptimierungsmodus ferner ein Aufrechterhalten eines aktuellen Vakuumdrucks für eine Druckaufrechterhaltungszeitdauer als Reaktion darauf aufweist, dass der zweite Pumpenarbeitszykluswert kleiner als der zuvor bestimmte Arbeitszyklusschwellenwert ist, wobei die Steuerung (110) optional konfiguriert ist, um die NPWT-Vorrichtung (100) von dem Druckoptimierungsmodus in den Standardtherapiemodus zu überführen, als Reaktion darauf, dass der Pumpenarbeitszykluswert an einem Ende der Druckaufrechterhaltungszeitdauer kleiner als der zuvor bestimmte Arbeitszyklusschwellenwert ist.

**14.** Die NPWT-Vorrichtung nach Anspruch 1, wobei die Steuerung (110) konfiguriert ist zum:

Bestimmen einer Differenz zwischen dem überwachten tatsächlichen Vakuumdruck und einem Mindestschwellenvakuumdruck; und
Überführen der NPWT-Vorrichtung (100) von dem Druckoptimierungsbetriebsmodus in den Druckerhaltungsbetriebsmodus basierend auf der bestimmten Differenz zwischen dem überwachten tatsächlichen Vakuumdruck und dem Mindestschwellenvakuumdruck.

**15.** Die NPWT-Vorrichtung nach Anspruch 1, wobei der Erhaltungsbetriebsmodus ferner ein wiederholtes Reduzieren des Sollvakuumdrucks aufweist, bis der Sollvakuumdruck im Wesentlichen gleich dem absoluten Mindestdruck ist, wobei der Erhaltungsbetriebsmodus optional das Betreiben der Pumpe (142) aufweist, um den absoluten Mindestdruck intermittierend zu erzeugen.

**Revendications**

**1.** Dispositif de traitement de plaie par pression négative, NPWT (100) configuré pour effectuer un NPWT, le dispositif NPWT (100) comprenant :

une batterie (120) configurée pour alimenter le dispositif NPWT (100) en énergie ;
une pompe (142) configurée pour recevoir l'énergie de la batterie (120) et pour produire un vide à une pression de consigne pour effectuer le NPWT ;
une interface utilisateur (106) configurée pour fournir des alertes à un utilisateur ;
un dispositif de commande (110) configuré pour recevoir l'énergie de la batterie (120) et pour ajuster la pression de consigne de la pompe et configuré en outre pour :

faire fonctionner le dispositif NPWT (100) dans un mode de thérapie normalisé, dans lequel le mode de thérapie normalisé comprend le fonctionnement de la pompe (142) à une première pression à vide de consigne et la comparaison de manière périodique d'une valeur de cycle de travail déterminée de la pompe (142) avec une valeur de seuil de cycle de travail prédéterminée ;
faire fonctionner le dispositif NPWT (100) dans un mode de thérapie d'assistance à l'étanchéité, dans lequel le mode de thérapie d'assistance à l'étanchéité comprend le fonctionnement de la pompe (142) à une seconde pression à vide de consigne, dans lequel la seconde pression à vide de consigne est supérieure à la première pression à vide de consigne ;
**caractérisé en ce que** le dispositif de commande (110) est configuré pour faire fonctionner le dispositif NPWT (100) dans un mode d'optimisation de pression, dans lequel le mode d'optimisation de pression comprend :

la détermination d'une valeur de cycle de travail de pompe initiale et d'une capacité de batterie initiale ;
la réduction de la pression de consigne du vide par une quantité déterminée à une fin d'un pas de temps ;
la détermination d'une seconde valeur de cycle de travail de pompe à la fin du pas de temps ;
la surveillance d'une pression à vide réelle produite par la pompe (142) à la fin du pas de temps ;
la répétition des étapes de réduction de la pression de consigne, de calcul de la seconde valeur de cycle de travail de pompe, et de surveillance de la pression à vide réelle de la pompe (142) jusqu'à ce que la seconde valeur de cycle de travail de pompe soit inférieure à la valeur de seuil de cycle de travail prédéterminée ; et
dans lequel la quantité déterminée et le pas de temps sont déterminés en fonction d'au moins l'une parmi la valeur de cycle de travail de pompe initiale et la capacité de batterie initiale ; et
faire fonctionner le dispositif NPWT (100) dans un mode de fonctionnement de conservation, dans lequel le mode de fonctionnement de conservation comprend la réduction de la pression à vide de consigne à une pression minimale absolue.

**2.** Dispositif NPWT selon la revendication 1, dans lequel le dispositif de commande (110) est en outre configuré pour amener l'interface utilisateur (106) à fournir une alerte, dans lequel l'alerte indique au moins l'un parmi un événement de fuite et une faible capacité de batterie, dans lequel l'alerte comprend éventuellement au moins l'une parmi une alerte visuelle et une alerte auditive.

**3.** Dispositif NPWT selon la revendication 1, dans lequel le dispositif de commande (110) est en outre configuré pour déterminer une valeur de travail de pompe continue, dans lequel la valeur de travail de pompe continue garantit que le NPWT puisse être maintenu pendant une durée de thérapie, dans lequel la valeur de travail de pompe continue est éventuellement déterminée en fonction d'au moins l'une parmi les propriétés de la pompe (142), la capacité de batterie de la batterie (120), et de la durée de thérapie.

**4.** Dispositif NPWT selon la revendication 1, dans lequel le dispositif de commande (110) est en outre configuré pour faire transitionner le dispositif NPWT (100) du mode de thérapie normalisé au mode de thérapie d'assistance à l'étanchéité en réponse à la valeur de cycle de travail déterminée dépassant la valeur de seuil de cycle de travail prédéterminée.

**5.** Dispositif NPWT selon la revendication 1, dans lequel le mode de thérapie normalisé comprend en outre l'actionnement de manière périodique de la pompe (142) entre un état de marche et un état d'arrêt pour produire la première pression à vide de consigne.

**6.** Dispositif NPWT selon la revendication 1, dans lequel le mode de thérapie d'assistance à l'étanchéité comprend en outre l'augmentation de manière répétée de la pression de consigne de la pompe (142) jusqu'à ce que la pompe atteigne la seconde pression à vide.

**7.** Dispositif NPWT selon la revendication 6, dans lequel le mode de thérapie d'assistance à l'étanchéité comprend en outre le réglage de la pompe (142) pour fonctionner à la première pression à vide pendant un laps de temps prédéterminé en réponse à la pompe atteignant la seconde pression à vide de consigne.

**8.** Dispositif NPWT selon la revendication 7, dans lequel le mode de thérapie d'assistance à l'étanchéité comprend en outre le calcul de la valeur de cycle de travail de pompe de la pompe (142) et la surveillance de la pression à vide réelle de la pompe à une fin du laps de temps prédéterminé.

**9.** Dispositif NPWT selon la revendication 8, dans lequel le dispositif de commande (110) est configuré pour faire transitionner le dispositif NPWT (100) du mode de thérapie d'assistance à l'étanchéité au mode de thérapie normalisé en réponse à la valeur de cycle de travail de pompe calculée de la pompe (142) à la fin du laps de temps prédéterminé qui est inférieure à la valeur de seuil de cycle de travail prédéterminée.

**10.** Dispositif NPWT selon la revendication 8, dans lequel le dispositif de commande (110) est configuré pour faire transitionner le dispositif NPWT (100) du mode de thérapie d'assistance à l'étanchéité au mode d'optimisation de pression en réponse à au moins l'une parmi :

la valeur de cycle de travail de pompe calculée de la pompe (142) à la fin du laps de temps prédéterminé qui est supérieure à la valeur de seuil de cycle de travail prédéterminée ; et
la dépression réelle surveillée de la pompe (142) à la fin du laps de temps prédéterminé qui est inférieure à la seconde pression à vide de consigne.

**11.** Dispositif NPWT selon la revendication 1, dans lequel le mode d'optimisation de pression comprend en outre :

l'augmentation de la pression à videde consigne par une quantité prédéterminée ;
le calcul du cycle de travail de pompe ; et
la répétition des étapes d'augmentation de la pression à vide de consigne et de calcul du cycle de travail de pompe jusqu'à ce que le cycle de travail de pompe calculé soit sensiblement égal à la valeur de seuil de cycle de travail prédéterminée.

**12.** Dispositif NPWT selon la revendication 1, dans lequel le mode d'optimisation de pression comprend en outre le fait d'amener l'interface utilisateur (106) à afficher une alerte de fuite en réponse à la chute de pression à vide réelle surveillée en dessous d'une pression à vide minimale autorisée.

**13.** Dispositif NPWT selon la revendication 1, dans lequel le mode d'optimisation de pression comprend en outre le maintien d'une pression à vide actuelle pendant un laps de temps de maintien de pression en réponse à la seconde valeur de cycle de travail de pompe qui est inférieure à la valeur de seuil de cycle de travail prédéterminée, dans lequel le dispositif de commande (110) est éventuellement configuré pour faire transitionner le dispositif NPWT (100) du mode d'optimisation de pression au mode de thérapie normalisé en réponse à la valeur de cycle de travail de pompe à une fin du laps de temps de maintien de pression qui est inférieure à la valeur de seuil de cycle de travail prédéterminée.

**14.** Dispositif NPWT selon la revendication 1, dans lequel le dispositif de commande (110) est configuré pour :

déterminer une différence entre la pression à vide réelle surveillée et une pression à vide de seuil minimale ; et

faire transitionner le dispositif NPWT (100) du mode de fonctionnement d'optimisation de pression au mode de fonctionnement de conservation en fonction de la différence déterminée entre la pression à vide réelle surveillée et la pression à vide de seuil minimale.

**15.** Dispositif NPWT selon la revendication 1, dans lequel le mode de fonctionnement de conservation comprend en outre la réduction de manière répétée de la pression à vide de consigne jusqu'à ce que la pression à vide de consigne soit sensiblement égale à la pression minimale absolue, dans lequel le mode de fonctionnement de conservation comprend éventuellement le fonctionnement de la pompe (142) pour produire de manière intermittente la pression minimale absolue.

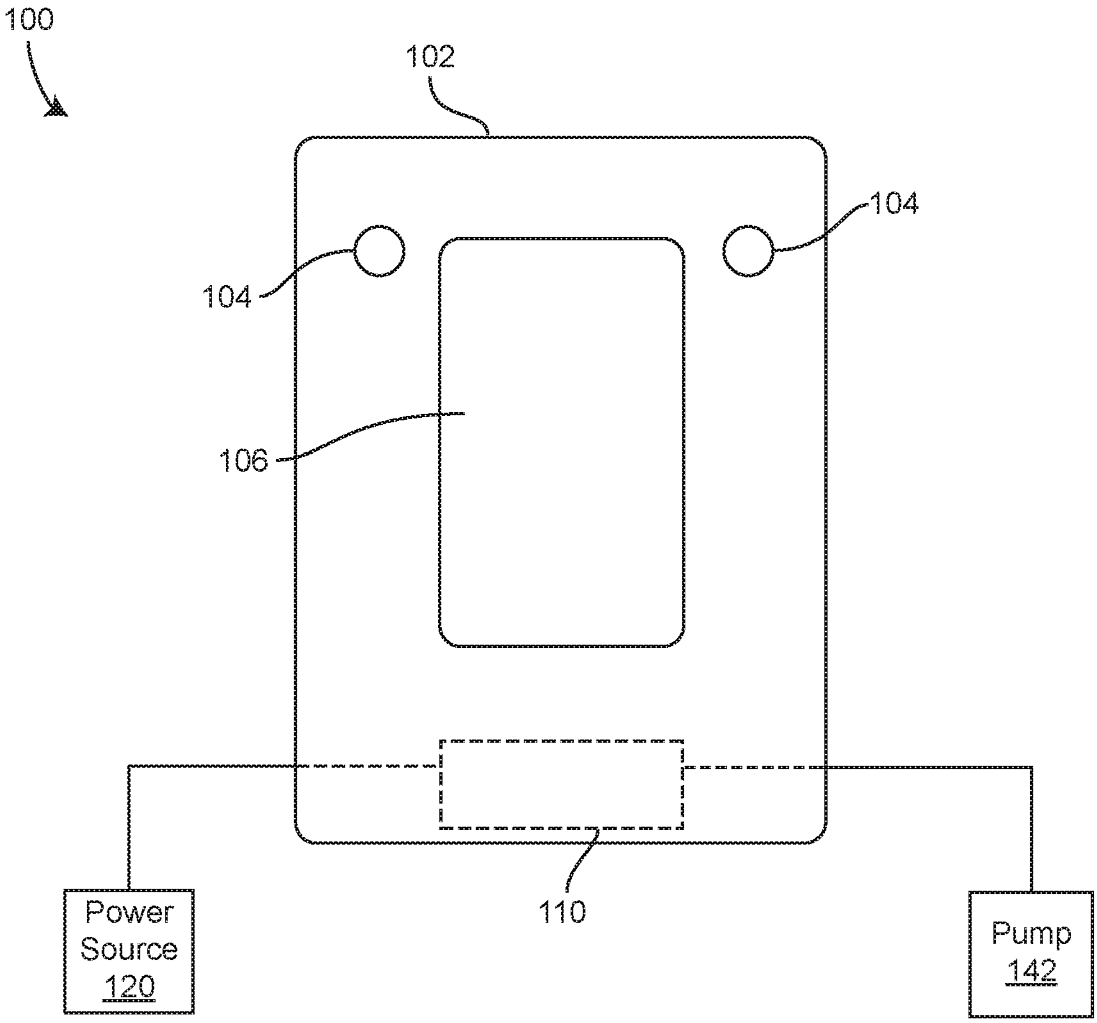


FIG. 1

FIG. 2

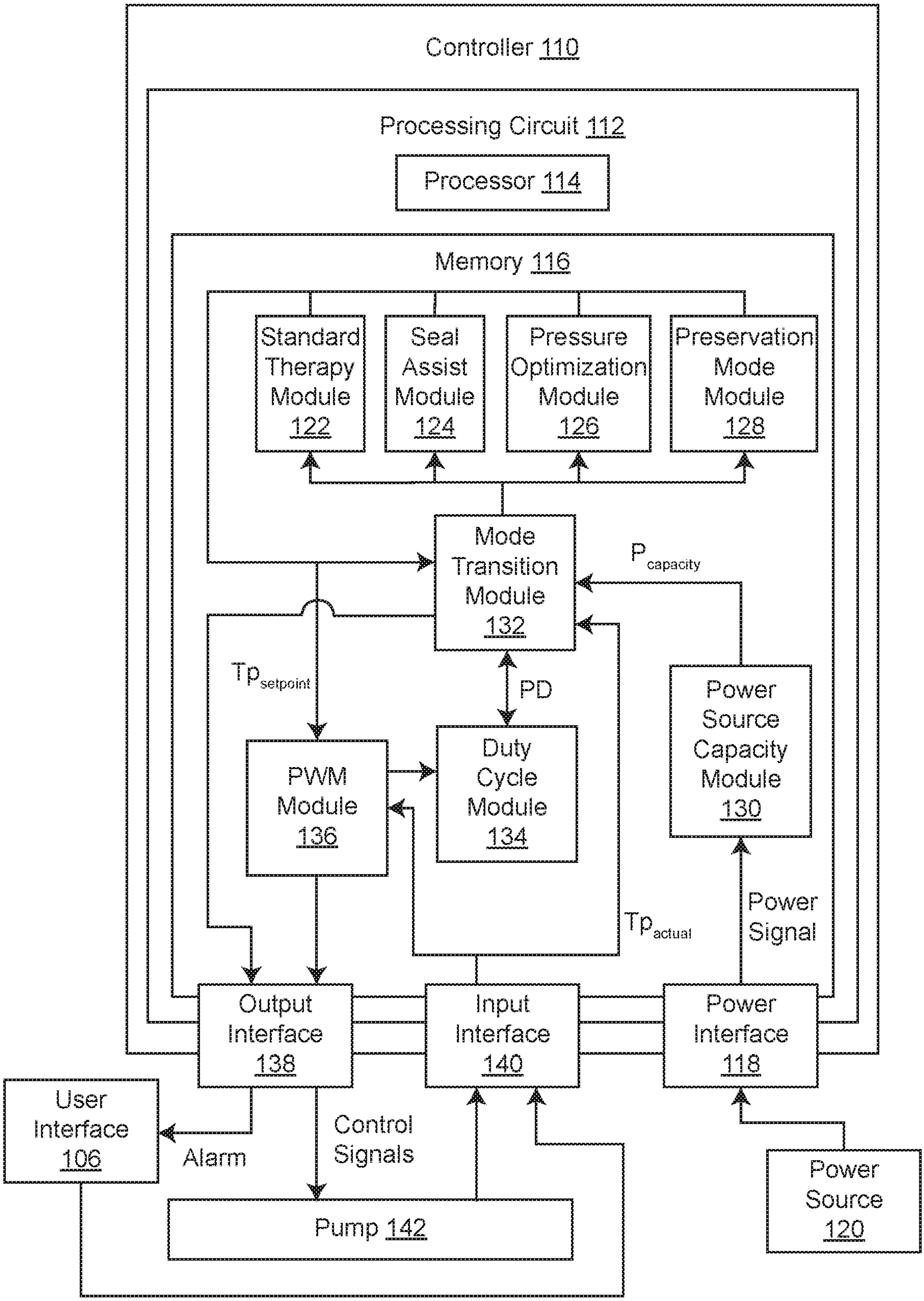
Controller 110
Processing Circuit 112
Processor 114
Memory 116
Standard Therapy Module 122
Seal Assist Module 124
Pressure Optimization Module 126
Preservation Mode Module 128
Mode Transition Module 132
P capacity
Tp setpoint
PD
Power Source Capacity Module 130
PWM Module 136
Duty Cycle Module 134
Tp actual
Power Signal
Output Interface 138
Input Interface 140
Power Interface 118
User Interface 106
Alarm
Control Signals
Pump 142
Power Source 120

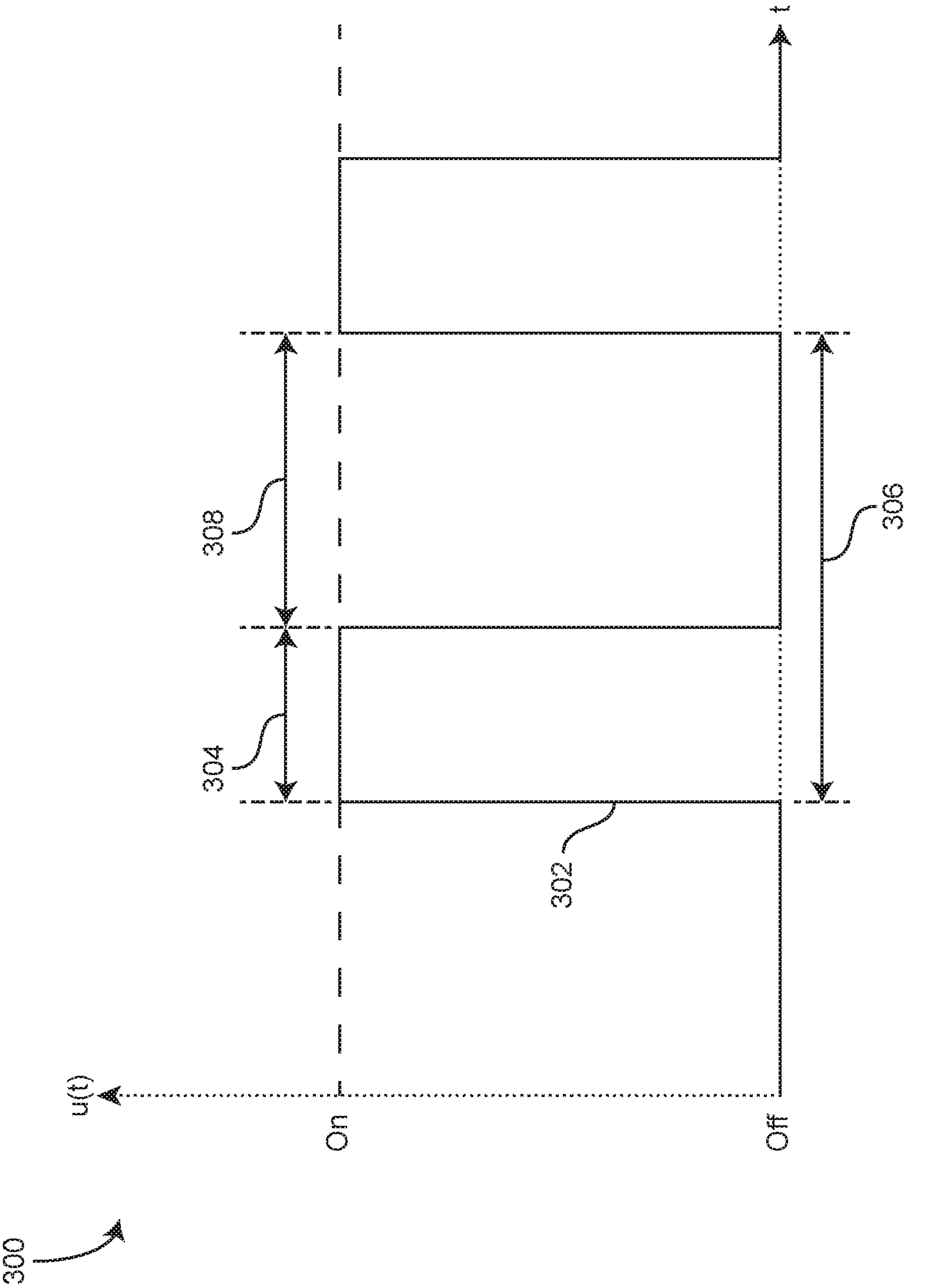


FIG. 3

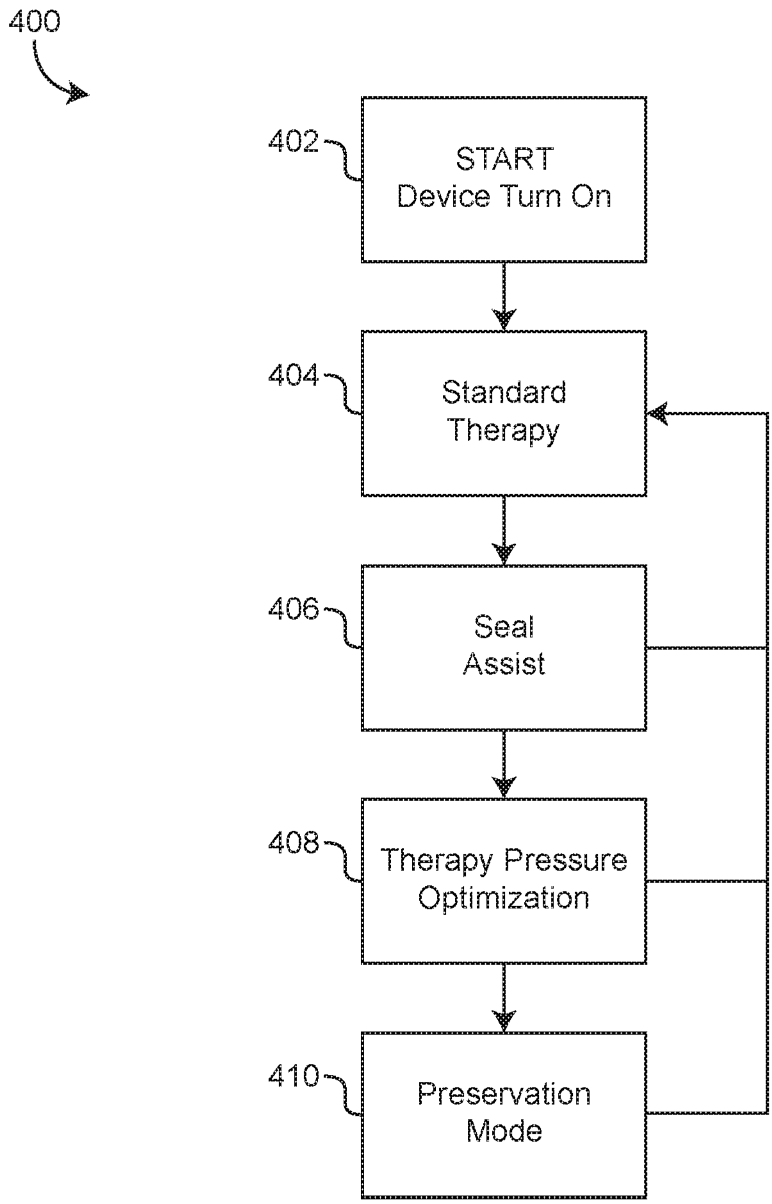
400
402
START
Device Turn On
404
Standard
Therapy
406
Seal
Assist
408
Therapy Pressure
Optimization
410
Preservation
Mode

FIG. 4

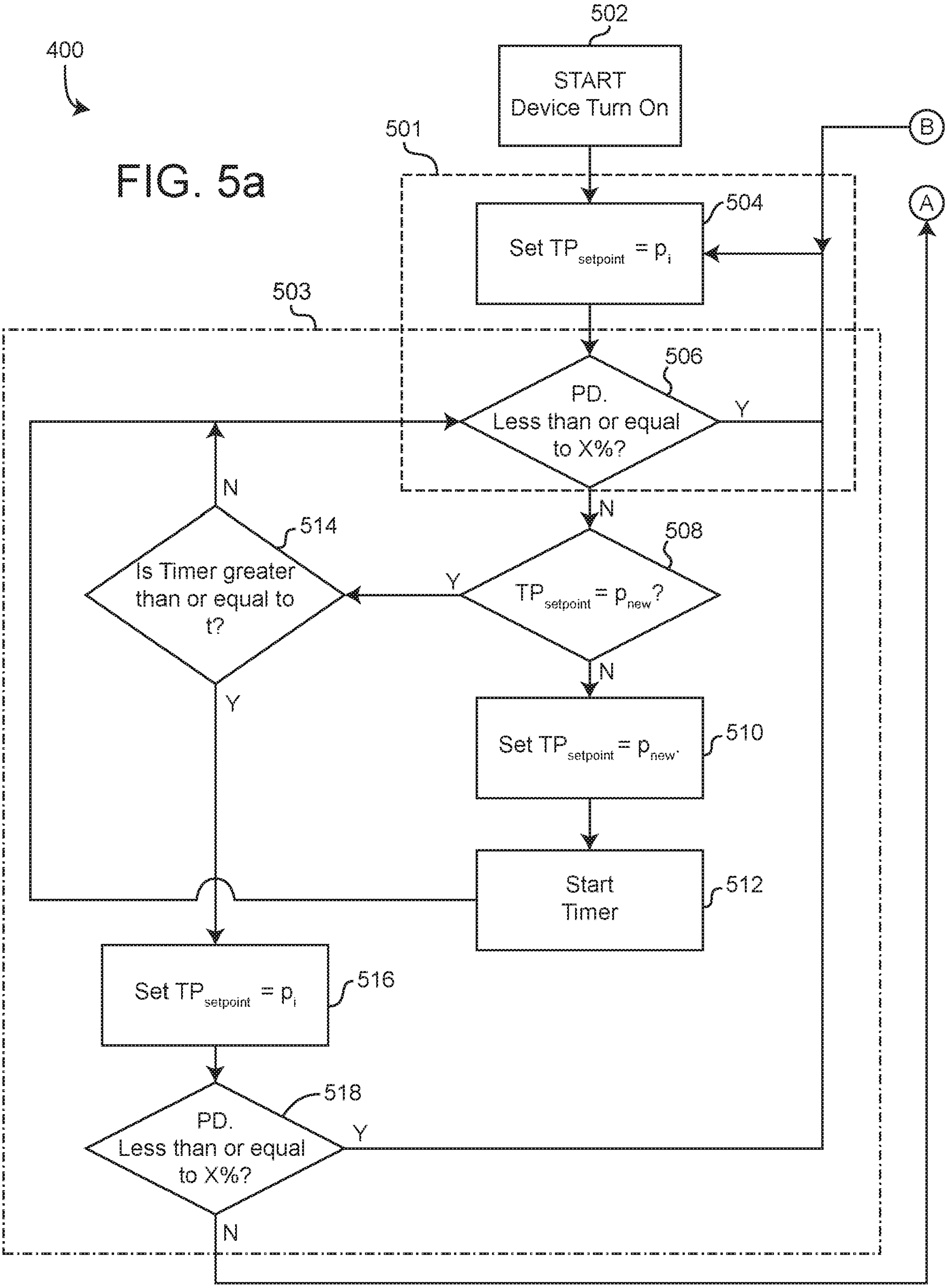
400
502
START
Device Turn On
501
504
Set TP setpoint = pi
503
506
PD.
Less than or equal
to X%?
Y
N
514
Is Timer greater
than or equal to
t?
Y
508
TP setpoint = pnew?
N
510
Set TP setpoint = pnew.
512
Start
Timer
516
Set TP setpoint = pi
518
PD.
Less than or equal
to X%?
Y
N
A
B

FIG. 5a

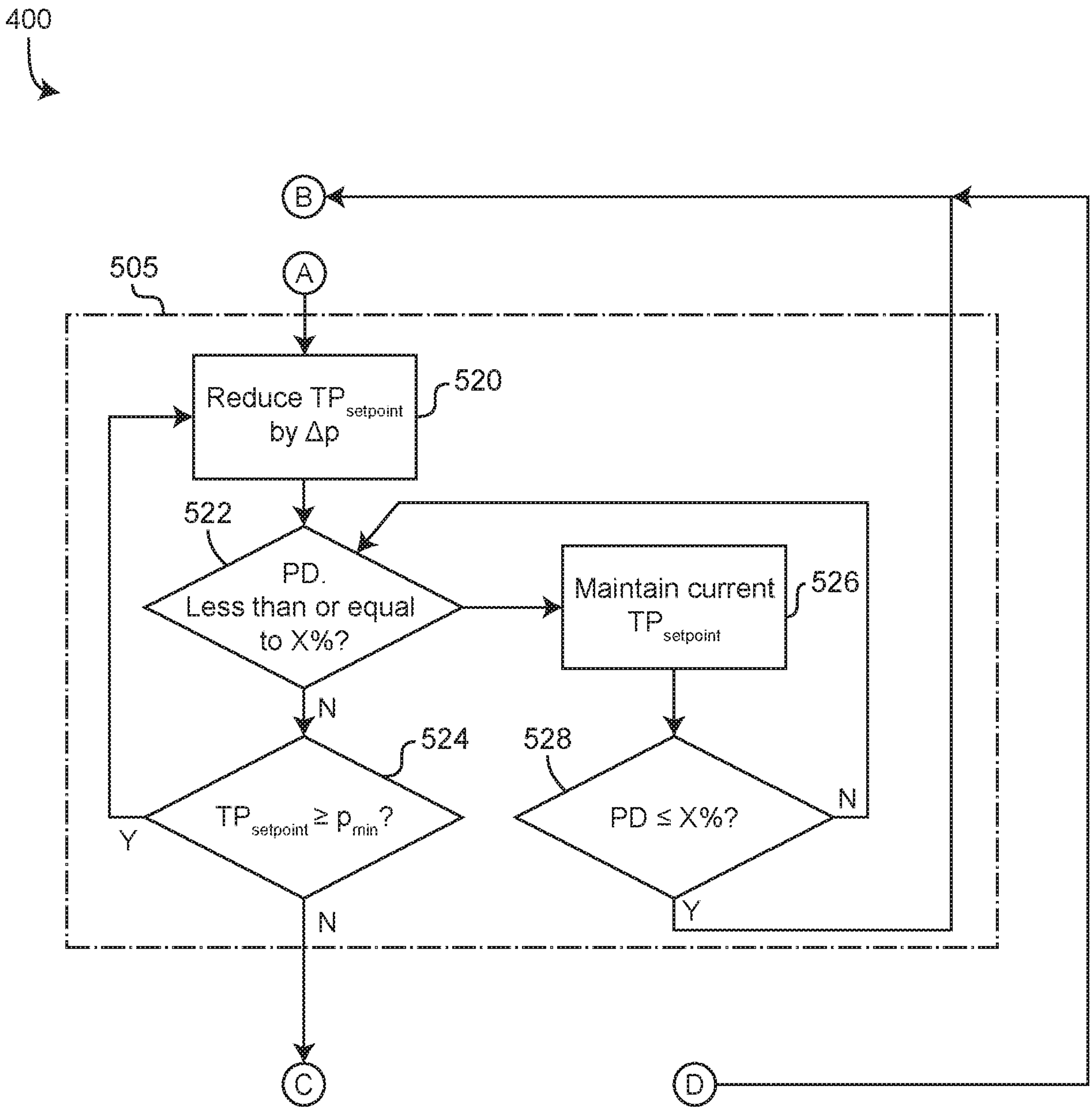
400
B
505
A
Reduce $TP_{setpoint}$ by Δp
520
522
PD. Less than or equal to X%?
Maintain current $TP_{setpoint}$
526
N
524
528
$TP_{setpoint} \geq p_{min}$?
PD ≤ X%?
Y
N
N
Y
C
D

FIG. 5b

FIG. 5c

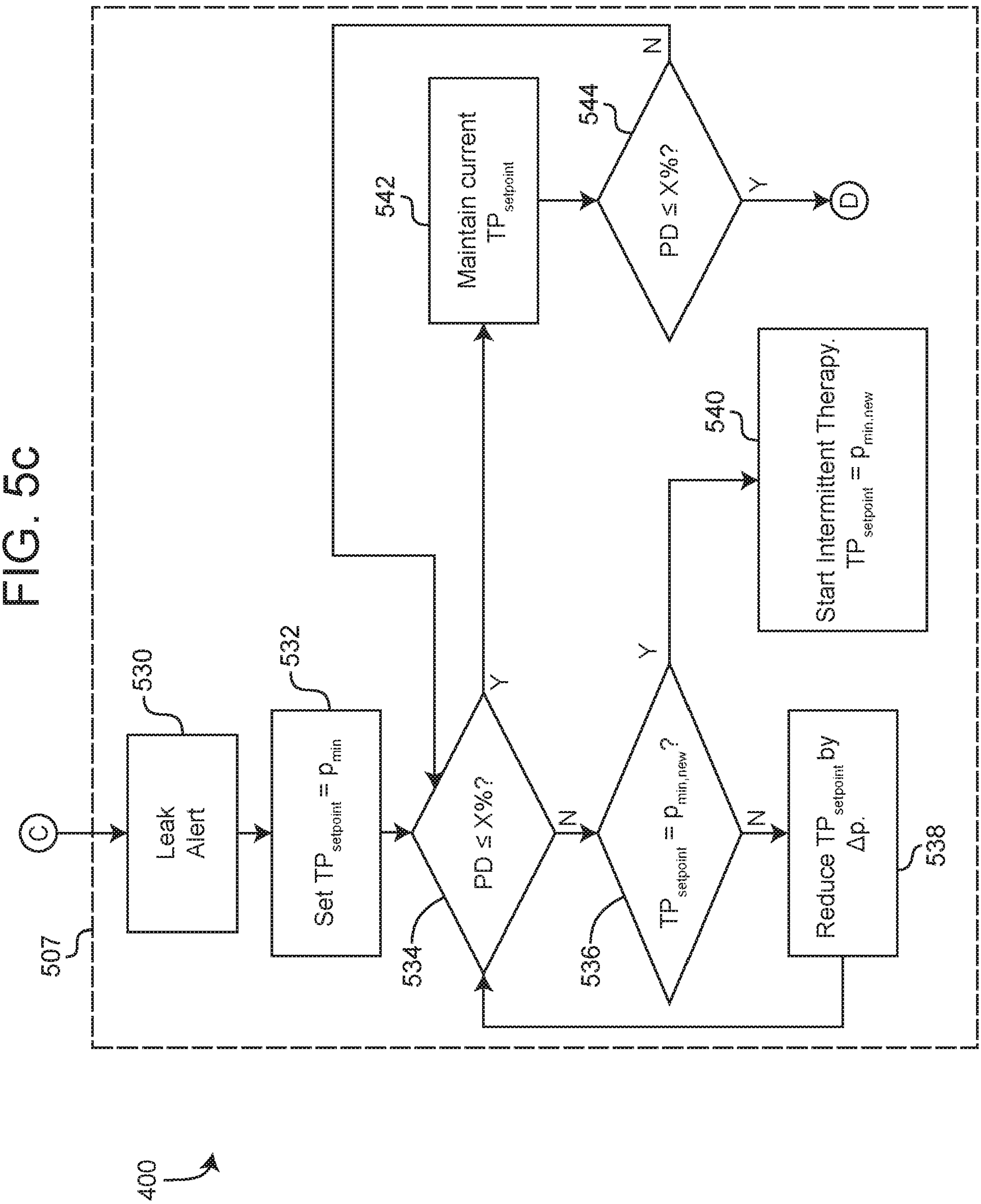
507
C
Leak Alert
530
Set $TP_{setpoint}$ = $p_{min}$
532
PD ≤ X%?
534
Y
N
$TP_{setpoint}$ = $p_{min,new}$?
536
Y
N
Reduce $TP_{setpoint}$ by Δp.
538
Start Intermittent Therapy. $TP_{setpoint}$ = $p_{min,new}$
540
Maintain current $TP_{setpoint}$
542
PD ≤ X%?
544
Y
N
D
400

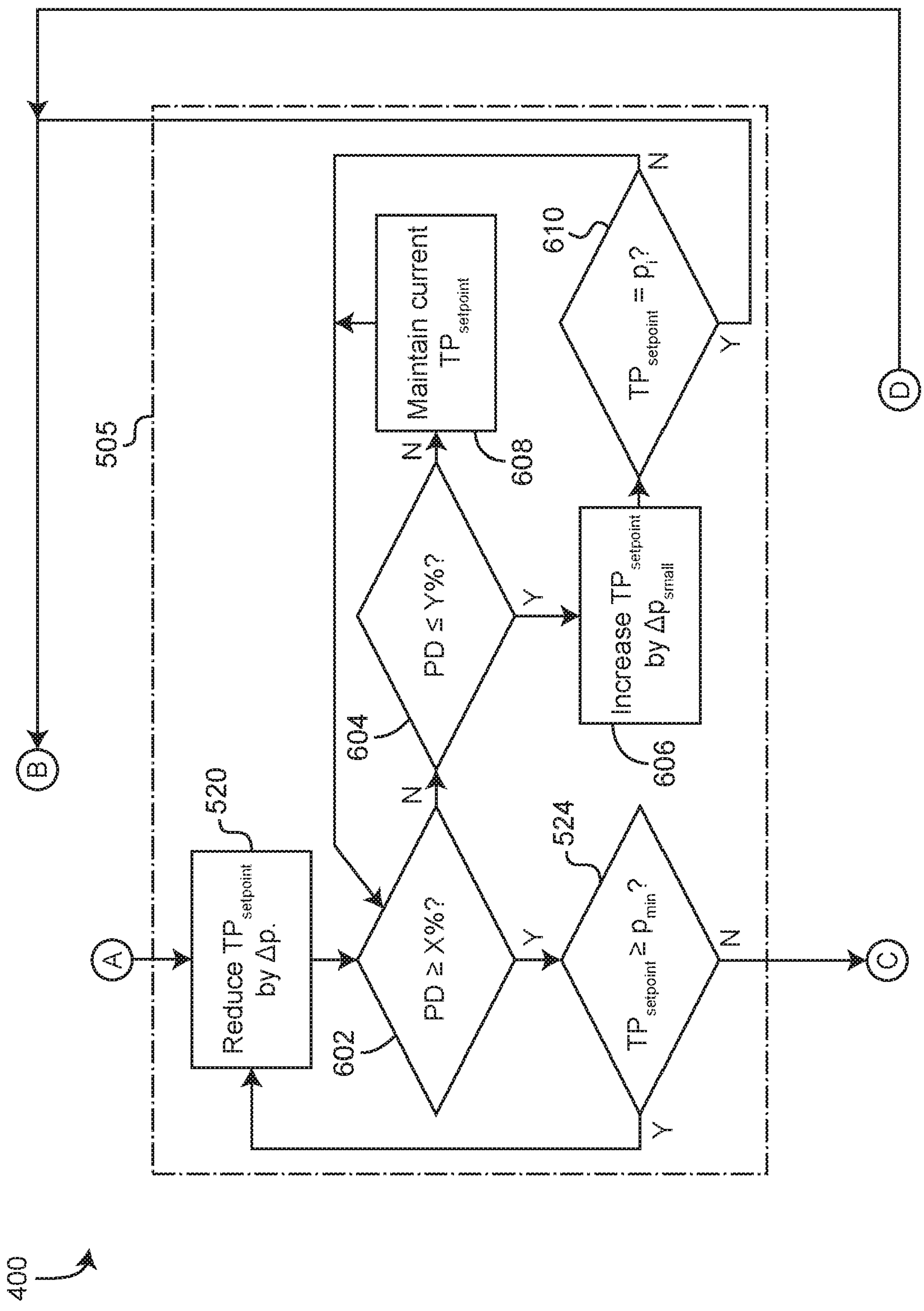
400
505
A
Reduce $TP_{setpoint}$ by $\Delta p$.
520
PD ≥ X%?
602
Y
N
$TP_{setpoint} \geq p_{min}$?
524
Y
N
C
PD ≤ Y%?
604
Y
N
Maintain current $TP_{setpoint}$
608
Increase $TP_{setpoint}$ by $\Delta p_{small}$
606
$TP_{setpoint} = p_i$?
610
Y
N
B
D

FIG. 6a

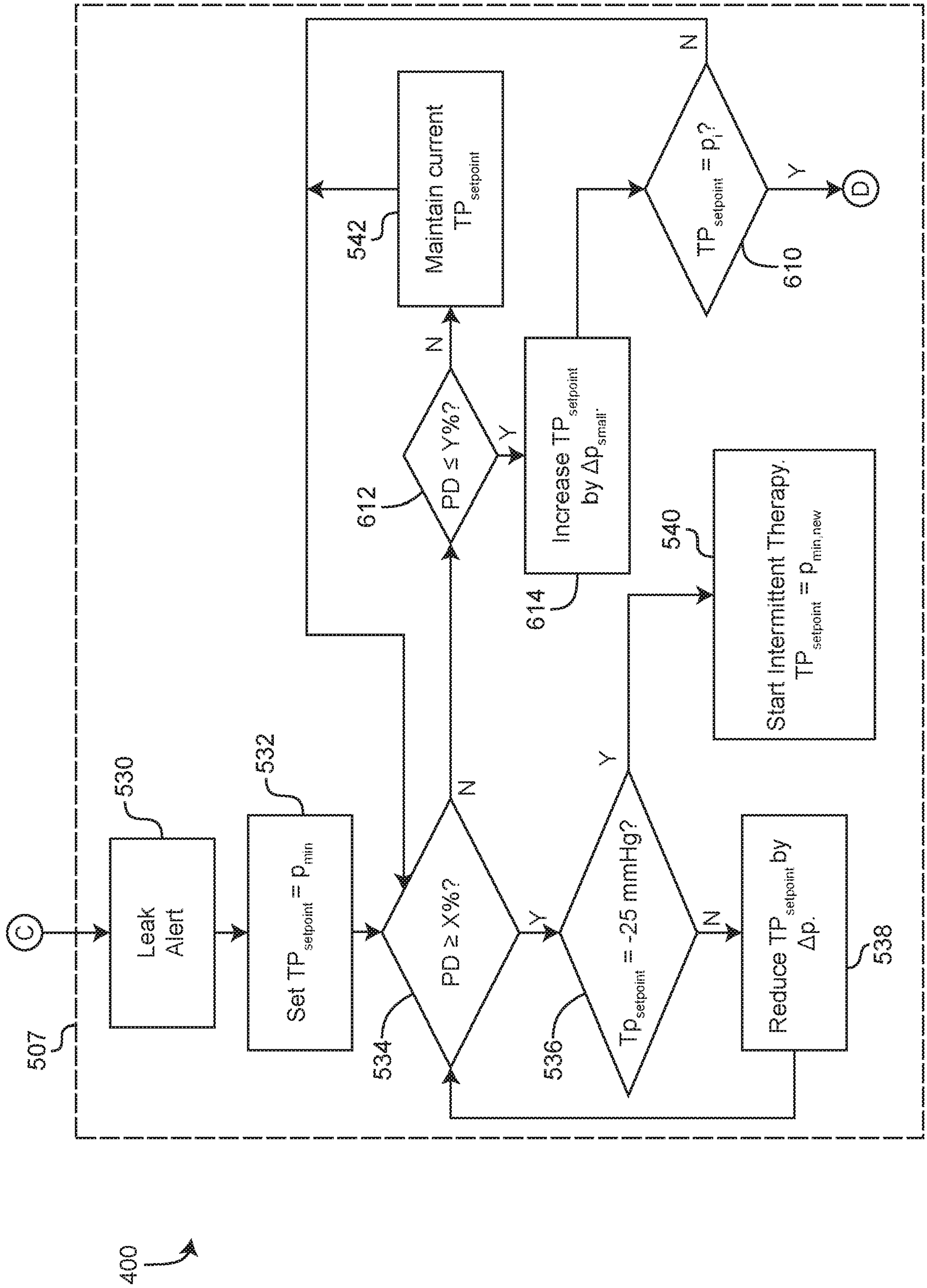
400
507
C
Leak Alert
530
Set $TP_{setpoint} = p_{min}$
532
PD ≥ X%?
534
Y
N
$Tp_{setpoint}$ = -25 mmHg?
536
Y
N
Reduce $TP_{setpoint}$ by Δp.
538
Start Intermittent Therapy. $TP_{setpoint} = p_{min,new}$
540
PD ≤ Y%?
612
Y
N
Increase $TP_{setpoint}$ by $Δp_{small}$.
614
Maintain current $TP_{setpoint}$
542
$TP_{setpoint} = p_i$?
610
Y
N
D

FIG. 6b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018041854 A1
- WO 2013140255 A1